(19) Europäisches Patentamt
European Patent Office
Office européen des brevets

(11) EP 0 729 972 A1

(12) **EUROPÄISCHE PATENTANMELDUNG**

(43) Veröffentlichungstag: **04.09.1996 Patentblatt 1996/36**

(51) Int. Cl.$^6$: **C07K 5/10**, C07K 7/06, C07K 7/08

(21) Anmeldenummer: **96102256.3**

(22) Anmeldetag: **15.02.1996**

(84) Benannte Vertragsstaaten:
**AT BE CH DE DK ES FR GB IT LI NL**

(30) Priorität: **28.02.1995 CH 567/95**

(71) Anmelder: **F. HOFFMANN-LA ROCHE AG
4070 Basel (CH)**

(72) Erfinder:
- **Abrecht, Christine
2543 Lengnau (CH)**
- **Grieder, Alfred
4450 Sissach (CH)**
- **Müller, Klaus
4142 Münchenstein (CH)**
- **Obrecht, Daniel
CH-4055 Basel (CH)**

(74) Vertreter: **Braun, Axel et al
F.Hoffmann-La Roche AG
Patent Department (PLP),
124 Grenzacherstrasse
4070 Basel (CH)**

(54) **Tetrahydronaphthalin-Peptidderivate**

(57) Tetrahydronaphthalinderivate der allgemeinen Formeln

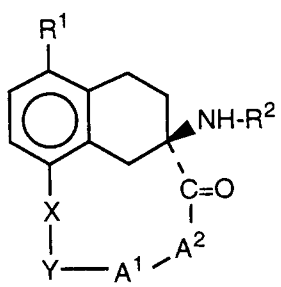

Ia und

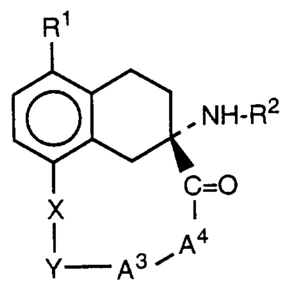

Ib

worin

| | |
|---|---|
| $R^1$ | Wasserstoff, Brom, Cyano, Formyl, Hydroxy, niederes Alkyl, niederes Alkenyl, niederes Alkinyl, niederes Alkoxy, Aryloxy, niederes Aralkoxy oder Aryl; |
| $R^2$ | eine gegebenenfalls geschützte Kette von bis zu 20 Aminosäureresten, deren N-terminale Aminosäure eine freie oder geschützte Aminogruppe aufweist; |
| $A^1$, $A^2$, $A^3$ und $A^4$ | je Reste von α-Aminosäuren, deren α-C-Atom, wenn es unsymmetrisch ist, in $A^1$ und $A^2$ in der L-Konfiguration und in $A^3$ und $A^4$ in der D-Konfiguration vorliegt; |
| X | Sauerstoff oder Schwefel; |

EP 0 729 972 A1

Printed by Rank Xerox (UK) Business Services
2.13.4/3.4

(Forts. nächste Seite)

Y einen Rest der Formel

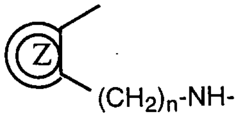

(a)

oder

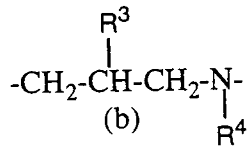

(b)

n die Zahl 0 oder 1;

$R^3$ und $R^4$ je Wasserstoff oder niederes Alkyl; und

Z zusammen mit den beiden C-Atomen einen gegebenenfalls substituierten Benzol-, Furan-, Thiophen-, Pyridin- oder Pyrimidinring bedeuten;

sowie Salze davon, eignen sich als Mimetica von helicalen Domänen von Proteinen, um deren Rolle bezüglich der Wechselwirkungen mit anderen Proteinen oder mit DNA bzw. RNA aufzuklären. Sie eignen sich deshalb als Forschungshilfsmittel ("Research Tools"), um biologisch aktive Peptidsequenzen bereitzustellen.

## Beschreibung

Die vorliegende Erfindung betrifft Tetrahydronaphthalin-Derivate, und zwar in erster Linie Tetrahydronaphthalinderivate der allgemeinen Formeln

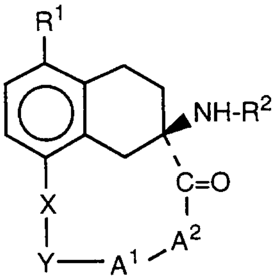 Ia und 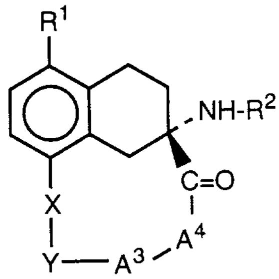 Ib

worin

| | |
|---|---|
| $R^1$ | Wasserstoff, Brom, Cyano, Formyl, Hydroxy, niederes Alkyl, niederes Alkenyl, niederes Alkinyl, niederes Alkoxy, Aryloxy, niederes Aralkoxy oder Aryl; |
| $R^2$ | eine gegebenenfalls geschützte Kette von bis zu 20 Aminosäureresten, deren N-terminale Aminosäure eine freie oder geschützte Aminogruppe aufweist; |
| $A^1$, $A^2$, $A^3$ und $A^4$ | je Reste von α-Aminosäuren, deren α-C-Atom, wenn es unsymmetrisch ist, in $A^1$ und $A^2$ in der L-Konfiguration und in $A^3$ und $A^4$ in der D-Konfiguration vorliegt; |
| X | Sauerstoff oder Schwefel; |
| Y | einen Rest der Formel |

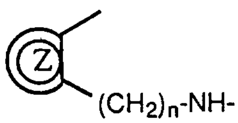
(a)
oder
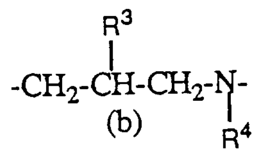
(b)

| | |
|---|---|
| n | die Zahl 0 oder 1; |
| $R^3$ und $R^4$ | je Wasserstoff oder niederes Alkyl; und |
| Z | zusammen mit den beiden C-Atomen einen gegebenenfalls substituierten Benzol-, Furan-, Thiophen-, Pyridin- oder Pyrimidinring bedeuten; |

sowie Salze davon.

Die Verbindungen der allgemeinen Formeln Ia und Ib und deren Salze sind neu. Diese Verbindungen und Salze, insbesondere diejenigen, welche keine Schutzgruppe(n) enthalten, sind wertvolle Hilfsmittel, um biologisch aktive Peptidsequenzen zu ermitteln und stellen somit sogenannte "Research Tools" dar; sie sind aber auch potentiell als Heilmittel geeignet.

Gegenstand der vorliegenden Erfindung sind die Verbindungen der allgemeinen Formeln Ia und Ib und Salze davon, deren Herstellung, Zwischenprodukte zu deren Herstellung und die Verwendung von Verbindungen der allgemeinen Formeln Ia und Ib und von Salzen davon als Research Tools.

Der Ausdruck "niederes Alkyl" umfasst geradkettige oder verzweigte gesättigte Kohlenwasserstoffreste mit bis zu 7, vorzugsweise bis zu 4 Kohlenstoffatomen, wie Methyl, Aethyl, n-Propyl, Isopropyl, n-Butyl, Isobutyl, tert.Butyl u.dgl. In analoger Weise umfassen die Ausdrücke "niederes Alkenyl" und "niederes Alkinyl" ungesättigte, eine Doppel- bzw. Dreifachbindung enthaltende Kohlenwasserstoffreste mit bis zu 7, vorzugsweise bis zu 4 Kohlenstoffatomen, wie Vinyl, Ethinyl, Allyl, Propargyl u.dgl. Der Ausdruck "niederes Alkoxy" umfasst Alkyloxygruppen im Sinne der obigen Umschreibung des Begriffs "niederes Alkyl". Der Ausdruck "Aryl" umfasst gegebenenfalls durch niederes Alkyl oder niederes Alkoxy mono- oder disubstituierte oder durch niederes Alkylendioxy substituierte Phenylreste. Der Ausdruck "Halogen" bezeichnet die vier Formen Fluor, Chlor, Brom und Jod, sofern nicht ausdrücklich anders angegeben.

Als Aminosäurereste kommen in erster Linie solche in Betracht, welche sich von α-Aminosäuren ableiten, insbesondere von natürlichen α-Aminosäuren; die Aminosäurereste können, sofern deren α-C-Atom unsymmetrisch ist, nicht nur in der L-Form, sondern auch in der D-Form vorliegen, und sie können gegebenenfalls geschützt sein. Nachstehend folgt eine Liste von Aminosäuren, welche bzw. deren Reste für die Zwecke der vorliegenden Erfindung geeignet sind, wobei die Abkürzungen den einschlägigen IUPAC-Regeln (Biochemistry 11, 1726 (1972)) und den allgemein üblichen Gepflogenheiten entsprechen.

| | |
|---|---|
| $Ac_3c$ | 1-Aminocyclopropancarbonsäure |
| $Ac_4c$ | 1-Aminocyclobutancarbonsäure |
| $Ac_5c$ | 1-Aminocyclopentancarbonsäure |
| $Ac_6c$ | 1-Aminocyclohexancarbonsäure |
| $Ac_7c$ | 1-Aminocycloheptancarbonsäure |
| Aib | 2-Amino-2-methylpropionsäure |
| Ala | L-Alanin |
| D-Ala | D-Alanin |
| β-Ala | β-Alanin |
| Arg | L-Arginin |
| D-Arg | D-Arginin |
| Asn | L-Asparagin |
| D-Asn | D-Asparagin |
| Asp | L-Asparaginsäure |
| D-Asp | D-Asparaginsäure |
| D-Asp (ONa) | Natrium-D-aspartat |
| $C_3al$ | L-3-cyclopropyalanin |
| $C_4al$ | L-3-cyclobutylalanin |
| $C_5al$ | L-3-cyclopentylalanin |
| $C_6al$ | L-3-cyclohexylalanin |
| Cys | L-Cystein |
| D-Cys | D-Cystein |
| Glu | L-Glutaminsäure |
| D-Glu | D-Glutaminsäure |
| Gln | L-Glutamin |
| D-Gln | D-Glutamin |
| Gly | Glycin |
| His | L-Histidin |
| D-His | D-Histidin |
| Hyp | 4-Hydroxy-L-prolin |
| Ile | L-Isoleucin |
| alle | L-Alloisoleucin |
| D-Ile | D-Isoleucin |
| D-alle | D-Alloisoleucin |
| D-Itg | D-2-(Isothiazolyl)glycin |
| Leu | L-Leucin |
| D-Leu | D-Leucin |
| tert.-Leu | L-2-Amino-3,3-dimethylbuttersäure |
| D-tert.-Leu | D-2-Amino-3,3-dimethylbuttersäure |
| Lys | L-Lysin |
| D-Lys | D-Lysin |
| Lys(CHO) | $N^6$-Formyl-L-lysin |
| MeAla | N-Methyl-L-alanin |
| MeLeu | N-Methyl-L-leucin |
| MeMet | N-Methyl-L-methionin |
| Met | L-Methionin |
| D-Met | D-Methionin |
| Met(O) | L-Methionin-sulfoxid |
| D-Met(O) | D-Methionin-sulfoxid |
| $Met(O_2)$ | L-Methionin-sulfon |
| $D-Met(O_2)$ | D-Methionin-sulfon |
| Nal | L-3-(1-Naphthylalanin) |
| D-Nal | D-3-(1-Naphthylalanin) |
| Nle | L-Norleucin |
| D-Nle | D-Norleucin |

| | |
|---|---|
| Nva | L-Norvalin |
| D-Nva | D-Norvalin |
| Orn | L-Ornithin |
| D-Orn | D-Ornithin |
| Orn(CHO) | $N^5$-Formyl-L-ornithin |
| Phe | L-Phenylalanin |
| D-Phe | D-Phenylalanin |
| L-Phg | L-Phenylglycin |
| D-Phg | D-Phenylglycin |
| Pip | L-Pipecolinsäure |
| D-Pip | D-Pipecolinsäure |
| Pro | L-Prolin |
| D-Pro | D-Prolin |
| Sar | Sarcosin |
| Ser | L-Serin |
| D-Ser | D-Serin |
| Thr | L-Threonin |
| D-Thr | D-Threonin |
| Thz | L-Thiazolidin-4-carbonsäure |
| D-Thz | D-Thiazolidin-4-carbonsäure |
| Trp | L-Tryptophan |
| D-Trp | D-Tryptophan |
| D-Trp(CHO) | $N^{in}$-Formyl-D-tryptophan |
| D-Trp(O) | D-3-(2,3-Dihydro-2-oxoindol-3-yl)alanin |
| Tyr | L-Tyrosin |
| D-Tyr | D-Tyrosin |
| Tza | L-3-(2-Thiazolyl)alanin |
| D-Tza | D-3-(2-Thiazolyl)alanin |
| Tzg | L-2-(Thiazolyl)glycin |
| D-Tzg | D-2-(Thiazolyl)glycin |
| Val | L-Valin |
| D-Val | D-Valin |

Geeignete Schutzgruppe für Aminosäuren bzw. deren Reste sind beispielsweise

- für die Aminogruppe (wie sie z.B. auch in der Seitenkette von Lysin vorliegt)

| | |
|---|---|
| Z | Benzyloxycarbonyl |
| Boc | tert.- Butyloxycarbonyl |
| Fmoc | Fluoren-9-ylmethoxycarbonyl |
| Alloc | Allyloxycarbonyl |
| Teoc | Trimethylsilyläthoxycarbonyl |
| Tcc | Trichloräthoxycarbonyl |
| Nps | o-Nitrophenylsulfenyl |
| | p-Brombenzoyl; |

- für die Carboxylgruppe (wie sie z.B. auch in der Seitenkette von Asparginsäure und Glutaminsäure vorliegt) durch Ueberführung in entsprechende Ester mit den Alkoholkomponenten

| | |
|---|---|
| tBu | tert.-Butyl |
| Bzl | Benzyl |
| Me | Methyl |
| Ph | Phenyl |
| Pac | Phenacyl |
| | Allyl |
| | Trimethylsilyläthyl |
| | Trichloräthyl; |

- für die Guanidingruppe (wie sie beispielsweise in der Seitenkette von Arginin vorliegt)

Pmc 2,2,5,7,8,-Pentamethylchroman-6-sulfonyl
Ts Tosyl
Z Benzyloxycarbonyl;

- für die Hydroxygruppe (wie sie beispielsweise in der Seitenkette von Threonin und Serin vorliegt)

tBu tert.-Butyl
Bzl Benzyl
Trityl;

- und für die Mercaptogruppe (wie sie beispielsweise in der Seitenkette von Cystein vorliegt)

tBu tert.-Butyl
Bzl Benzyl
Trityl
2-Methoxytrityl.

Wenn $R^1$ Aryl bedeutet, dann handelt es sich zweckmässigerweise um einen durch niederes Alkyl oder niederes Alkoxy mono- oder disubstituierten oder einen durch niederes Alkylendioxy substituierten Phenylrest. $R^2$ enthält zweckmässigerweise 9 bis 15 Aminosäurereste, insbesondere 9, 12 oder 15 Aminosäurereste. In erster Linie kommen Reste von $\alpha$-Aminosäuren in Betracht, deren $\alpha$-C-Atom, wenn es asymmetrisch ist, vorzugsweise in der L-Konfiguration vorliegt. Beispielsweise können die in $R^2$ enthaltenen Aminosäurereste von L-Alanin, L-Glutamin, L-Glutaminsäure, L-Isoleucin, L-Leucin, L-Lysin oder 2-Amino-2-methylpropionsäure abgeleitet sein. Wenn die Aminogruppe der N-terminalen Aminosäure im Rest $R^2$ geschützt ist, so kann die Schutzgruppe beispielsweise eine Acylgruppe, wie p-Brombenzoyl, sein. Zweckmässigerweise bedeuten $A^1$ bzw. $A^3$ einen Rest von L- bzw. D-Alanin, Asparagin, Asparaginsäure, Glutamin, Glutaminsäure oder Lysin oder von 2-Amino-2-methylpropionsäure und $A^2$ bzw. $A^4$ einen Rest von L- bzw. D-Alanin, Asparagin, Asparaginsäure, Glutamin, Glutaminsäure oder Lysin. Vorzugsweise bedeuten $A^1$ bzw. $A^3$ L- bzw. D-Alanyl und $A^2$ bzw. $A^4$ L- bzw. D-Alanyl.

Wenn der durch Z bezeichnete Benzol-, Furan-, Thiophen-, Pyridin- oder Pyrimidinring substituiert ist, so ist er zweckmässigerweise durch Nitro, Amino, niederes Alkanoylamino, Hydroxy, niederes Alkoxy, niederes Alkanoyloxy, niederes Alkyl, Fluor, Cyano, Carboxy oder Formyl mono- oder disubstituiert oder mit einem Benzolring zu einem bicyclischen System kondensiert.

Vorzugsweise bedeutet Y einen Rest der Formel (a), worin n die Zahl 0 und Z zusammen mit den beiden C-Atomen einen Benzolring bedeuten.

Repräsentative Verbindungen der Formeln Ia und Ib im Rahmen der vorliegenden Erfindung sind:

N-(4-Brom-benzoyl)-L-alanyl-L-alanyl-2-methyl-alanyl-L-alanyl-L-alanyl-L-alanyl-L-alanyl-L-alanyl-L-alanin (12S,15S,18R)-(12,15-dimethyl-11,14,17-trioxo-10,11,12,13,14,15,16,17,18,19,20,21-dodecahydro-1,18-ethenodibenz[b,n][1,5,8,11]oxatriazacyclopentadecin-18-yl)-amid,
N-(4-Brom-benzoyl)-L-alanyl-L-alanyl-2-methyl-alanyl-L-alanyl-L-alanyl-L-alanyl-L-alanyl-L-alanyl-L-alanin (12S,15S,18R)-(12,15-dimethyl-11,14,17-trioxo-10,11,12,13,14,15,16,17,18,19,20,21-dodecahydro-1,18-ethenopyrido[2,3-b]benz[n][1,5,8,11]oxatriazacyclopentadecin-18-yl)-amid;
N-(4-Brom-benzoyl)-L-alanyl-L-alanyl-2-methyl-alanyl-L-alanyl-L-alanyl-L-alanyl-L-alanyl-L-alanyl-L-alanin (3S,7S,10S,13R)-(3,7,10-trimethyl-6,9,12-trioxo-3,4,5,6,7,8,9,10,11,12,13,14,19,20-tetradecahydro-13,15-etheno- 2H-1,5,8,11-benzoxatriazacyclohexadecin-13-yl)-amid;
N-(4-Brom-benzoyl)-L-alanyl-L-alanyl-2-methyl-alanyl-L-alanyl-L-alanyl-L-alanyl-L-alanyl-L-alanyl-L-alanin (3S,7S,10S,13R)-(5-ethyl-3,7,10-trimethyl-6,9,12-trioxo-13,15-etheno-3,4,5,6,7,8,9,10,11,12,13,14,19,20-tetradecahydro-2H-1,5,8,11-benzoxatriazacyclohexadecin-13-yl)-amid; und
N-(4-Brom-benzoyl)-L-alanyl-L-alanyl-2-methyl-alanyl-L-alanyl-L-alanyl-L-alanyl-L-alanyl-L-alanyl-L-alanin (13S,16S,19R)-(13,16-dimethyl-12,15,18-trioxo-11,12,13,14,15,16,17,18,19,20,21,22-dodecahydro-1,19-etheno-10H-dibenz[b,o][1,5,8,11]oxatriazacyclohexadecin-19-yl)-amid.

Die Verbindungen der Formeln Ia und Ib sowie deren Salze können erfindungsgemäss dadurch hergestellt werden, dass man

a) eine Verbindung der allgemeinen Formel

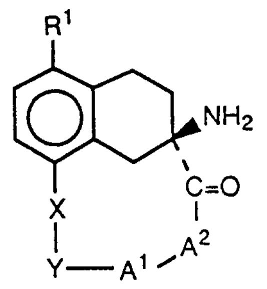 IIa bzw. 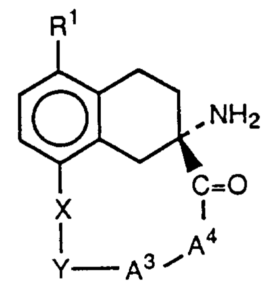 IIb

worin $R^1$, $A^1$, $A^2$, $A^3$, $A^4$, X und Y obige Bedeutung besitzen, in einem Schritt oder, vorzugsweise, in Etappen mit einer gegebenenfalls geschützten Kette von bis zu 20 Aminosäuren, deren N-terminale Aminosäure eine freie oder geschützte Aminogruppe aufweist, kuppelt; oder

b) eine Verbindung der allgemeinen Formel

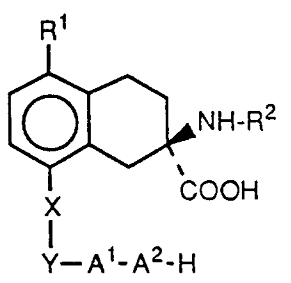 IIIa bzw. 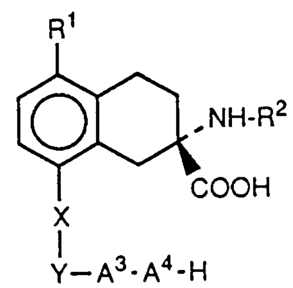 IIIb

worin $R^1$, $R^2$, $A^1$, $A^2$, $A^3$, $A^4$, X und Y obige Bedeutung besitzen, cyclisiert; oder

c) aus einer Verbindung der Formel Ia bzw. Ib, welche mindestens eine Schutzgruppe enthält, die Schutzgruppe(n) abspaltet; oder

d) eine Verbindung der Formel Ia bzw. Ib, welche ein basisches bzw. ein saures Zentrum enthält, mittels einer Säure bzw. einer Base in ein Salz überführt.

Die Verbindungen der Formeln IIa, IIb, IIIa und IIIb sind neu und ebenfalls Gegenstand der Erfindung.

Gemäss Verfahrensvariante a) wird die freie Aminogruppe einer Verbindung der Formel IIa bzw. IIb mit einer Peptidkomponente mit C-terminaler freier Carboxylgruppe gekuppelt, und bei der Cyclisation einer Verbindung der Formel IIIa bzw. IIIb werden eine freie Carboxylgruppe und eine freie Aminogruppe unter Bildung einer Amidbindung miteinander gekuppelt.

Zur Durchführung dieser Verfahrensvarianten werden in der Peptidchemie allgemein übliche und jedem Fachmann geläufige Methoden verwendet. Dabei kann auch die Methode der Festphasensynthese verwendet werden; als Träger eignen sich beispielsweise Polystyrol-Divinylbenzol u.dgl.

In Verfahrensvariante a) wird zweckmässigerweise in einer ersten Etappe mit einer einzigen Aminosäure gekuppelt und in einer zweiten Etappe der Rest der gewünschten Kette eingeführt.

Um die Kupplung gemäss Verfahrensvarianten a) und b) herbeizuführen, können alle möglichen in der Peptidchemie gebräuchlichen Aktivierungsreagenzien verwendet werden, wie z.B. O-Benzotriazol-1-yl-N,N,N',N'-tetramethyl-uroniuinhexafluorphosphat (HBTU); O-(1,2-Dihydro-2-oxo-1-pyridyl)-N,N,N',N'-tetramethyl-uroniumtetrafluoroborat (TPTU); 2-(1H-Benzotriazol-1-yl)-1,1,3,3-tetramethyluroniumtetrafluoroborat (TBTU); 1,1,3,3-Tetramethyl-2-[1H-1,2,3-triazolo[5,4-b]pyridin-1-yl]-uroniumtetrafluoroborat (TATU); 1-Hydroxybenzotriazol (HOBT) oder 3H-1,2,3-Triazolo[5,4-b]pyridin-1-ol (HOAT) in Kombination mit N,N-Dicyclohexylcarbodiimid u.dgl.

Durch Abspaltung der Schutzgruppe(n) aus einer mindestens eine Schutzgruppe enthaltenden Verbindung der Formel Ia bzw. Ib erhält man gemäss Verfahrensvariante c) eine entsprechende Verbindung der Formel Ia bzw. Ib, welche keine Schutzgruppe(n) enthält. Die Abspaltung der Schutzgruppe(n) erfolgt nach in der Peptidchemie allgemein üblichen und jedem Fachmann geläufigen Methoden, wobei natürlich jeweils die Natur der Schutzgruppe(n) in Betracht zu ziehen ist. So können die weiter oben genannten Schutzgruppen beispielsweise wie folgt abgespalten werden:

| | |
|---|---|
| Z : | Katalytische Hydrierung in Gegenwart von Pd/ C in einem niederen Alkanol, wie Methanol oder Aethanol. |
| Boc: | Mittels Trifluoressigsäure/ Methylenchlorid (1:1) oder mittels gesättigter Chlorwasserstofflösung in Essigsäureäthylester. |
| Fmoc : | Mittels Piperidin oder 1,8-Diazabicyclo[5.4.0]undec-7-en in Dimethylformamid. |
| Alloc : | Mittels Palladium-tetrakis-triphenylphosphin in Tetrahydrofuran/Dimethylsulfoxid / 0,1 N Salzsäure. |
| Teoc : | Mittels Caesiumfluorid oder Tetrabutylammoniumfluorid in Dimethylformamid o.dgl. |
| Tcc : | Mittels Zink in Eisessig oder Methanol. |
| Nps : | Mittels Natrium- oder Kaliumrhodanid in leicht saurem Milieu. |
| $^{t}$Bu : | Mittels Trifluoressigsäure/ Methylenchlorid (1:1). |
| Bzl : | Durch katalytische Hydrierung in Gegenwart von Pd / C in einem niederen Alkanol, wie Methanol oder Aethanol. |
| Me: | Mittels Lithiumhydroxid in Tetrahydrofuran/Methanol/Wasser (3:1:1). |
| Ph : | Mittels Natriumperoxyd bei pH 10,5. |
| Pac : | Mittels Zink in Eisessig oder Methanol oder mittels Natriumthiophenolat in Dimethylformamid. |
| Allyl : | Mittels Palladium-bis-triphenylphosphin-dichlorid und Tributylzinnhydrid oder mittels Palladium-tetrakis-triphenylphosphin in Tetrahydrofuran/ Dimethylsulfoxyd/0,5 N Salzsäure. |
| Trimethylsilyläthyl : | Mittels Caesiumfluorid oder Tetrabutylammoniumfluorid in Dimethylformamid o.dgl. |
| Trichloräthyl: | Mittels Zink in Eisessig oder Methanol. |
| Pmc : | Mittels wässriger Trifluoressigsäure. |
| Ts : | Mittels Natrium in flüssigem Ammoniak oder flüssigem Fluorwasserstoff. |

In analoger Weise kann eine an einem Polystyrol-Divinylbenzolharz o.dgl. durch Festphasensynthese hergestellte Verbindung der Formel Ia bzw. Ib von dem Trägerharz abgespalten werden, beispielsweise mittels "Field's Reagens", d.h. einer Mischung aus 82,5% Trifluoressigsäure, 5% Phenol. 5% Wasser, 5% Thioanisol und 2,5% 1,2-Aethandithiol.

Gemäss Verfahrensvariante d) kann eine Verbindung der Formel Ia bzw. Ib, welche ein basisches bzw. ein saures Zentrum enthält, mittels einer Säure bzw. einer Base in ein Salz übergeführt werden, was nach allgemein üblichen und jedem Fachmann geläufigen Methoden erfolgen kann. Als Säuren kann man hier für anorganische Säuren, wie Salzsäure, Schwefelsäure, Phosphorsäure o.dgl., oder organische Säuren, wie Trifluoressigsäure, Methansulfonsäure, p-Toluolsulfonsäure o.dgl., verwenden und als Base anorganischen Basen, wie Kaliumhydroxyd, Natriumhydroxyd o.dgl., oder organische Basen, wie Triaethylamin, Dimethylaminopyridin o.dgl.

Die für Verfahrensvarianten a) und b) benötigten Ausgangsprodukte der Formeln IIa bzw. IIb und IIIa bzw. IIIb können aus Verbindungen der allgemeinen Formeln

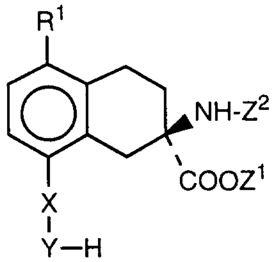 IVa bzw. 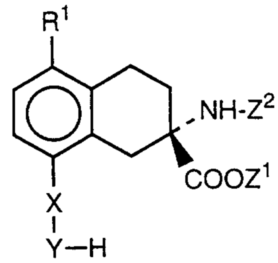 IVb

worin $R^1$, X und Y obige Bedeutung besitzen und $Z^1$ eine Carboxylschutzgruppe und $Z^2$ eine Aminoschutzgruppe bedeuten, hergestellt werden. Die Verbindungen der Formeln IVa und IVb sind neu und ebenfalls Gegenstand der vorliegenden Erfindung. Zweckmässigerweise bedeuten $Z^1$ Methyl, tert.-Butyl, Benzyl, Trimethylsilyläthyl oder Pentafluorphenyl und $Z^2$ Benzyloxycarbonyl, tert.-Butyloxycarbonyl oder 9-Fluorenylmethoxycarbonyl.

Die Herstellung der Verbindungen der Formeln IVa und IVb und deren Ueberführung in Verbindungen der Formen IIa bzw. IIb und IIIa bzw. IIIb werden nachstehend, z.T. anhand von Reaktionsschemata, näher erläutert.

## Reaktionsschema 1

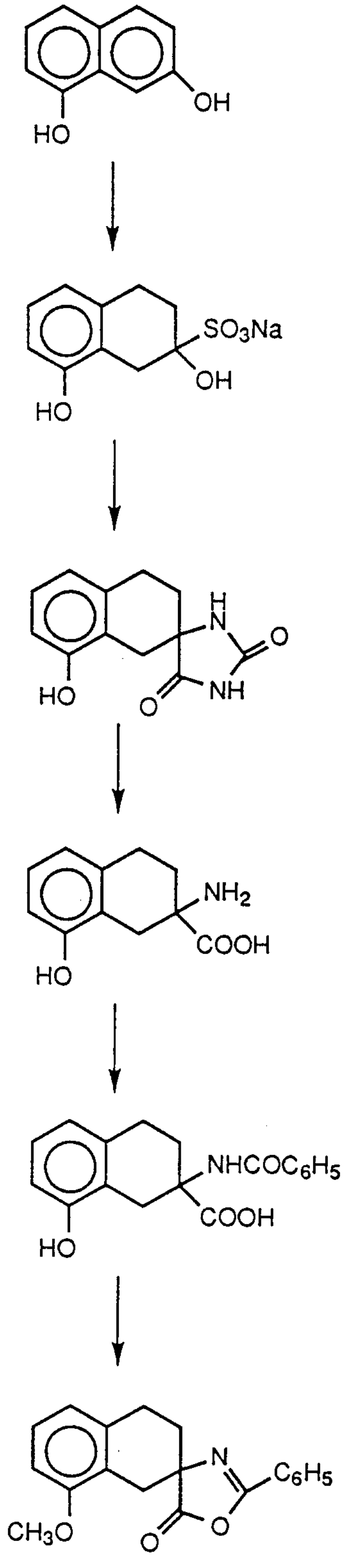

V

VI

VII

VIII

IX

X

## Reaktionsschema 2

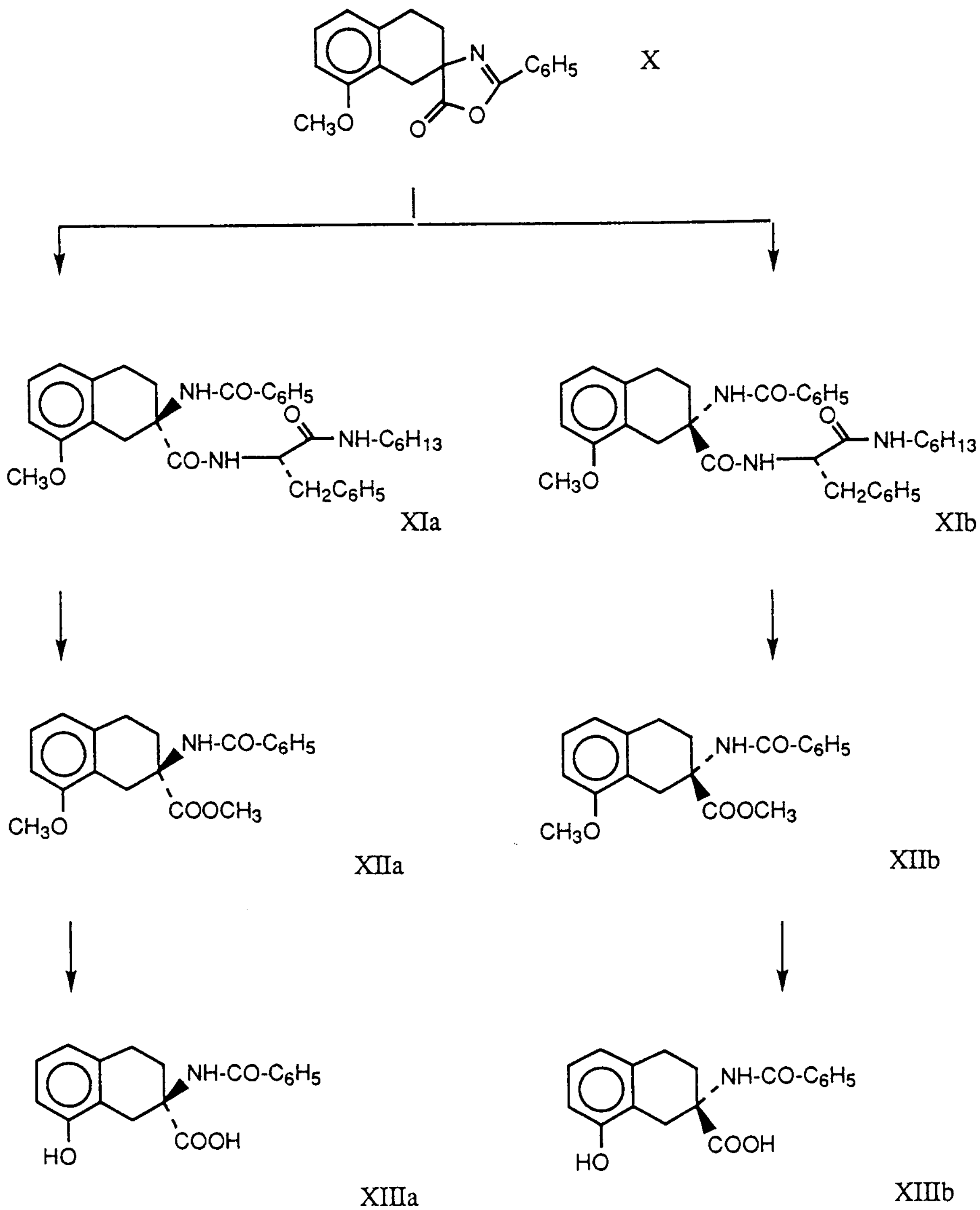

X

XIa

XIb

XIIa

XIIb

XIIIa

XIIIb

V → VI

1,7-Dihydroxynaphthalin (V) kann mittels Lithium in flüssigem Ammoniak/Tetrahydrofuran/tert.-Butanol in 8-Hydroxy-1,2,3,4-tetrahydronaphthalin-2-on übergeführt werden, welches - ohne dass es isoliert zu werden braucht -

mittels Natriumhydrogensulfit in (rac)-2,8-Dihydroxy-1,2,3,4-tetrahydronaphthalin-2-sulfonsäure-Natriumsalz (VI) übergeführt werden kann.

VI → VII

Das Natriumsalz der Formel VI kann mittels Kaliumcyanid und Ammoniumcarbonat in Aethanol/Wasser in die racemische Spiroverbindung der Formel VII übergeführt werden.

VII → VIII

Die Spiroverbindung der Formel VII kann durch Erhitzen mit Bariumhydroxid in Wasser und anschliessendes Ansäuern, z.B. mit wässeriger Schwefelsäure, in (rac)-2-Amino-8-hydroxy-1,2,3,4-tetrahydronaphthalin-2-carbonsäure (VIII) übergeführt werden.

VIII → IX

Die Verbindung der Formel VIII wird zunächst mittels Benzoylchlorid o.dgl. acyliert, zweckmässigerweise in Gegenwart eines säurebindenden Mittels, wie Natriumhydroxid o.dgl. Durch Behandlung des Acylierungsprodukts mit Natronlauge in Dioxan kann man dann zu (rac)-2-Benzamido-8-hydroxy-1,2,3,4-tetrahydronaphthalin-2-carbonsäure gelangen.

IX → X

Durch Behandlung der Verbindung der Formel IX mit Dicyclohexylcarbodiimid in Dichlormethan o.dgl. und anschliessende Methylierung, zweckmässigerweise mittels Dimethylsulfat in Gegenwart einer starken Base, wie Natriumhydrid, in Dioxan o.dgl. kann man zu (rac)-3,4-Dihydro-8-methoxy-2-phenylspiro[naphthalin-2(1H),4'(5'H)-oxazol]-5'-on gelangen.

X → XIa und XIb

Die Verbindung der Formel X kann mittels L-Phenylalanin-cyclohexylamid in ein Gemisch der beiden diastereoisomeren Verbindungen der Formeln XIa und XIb übergeführt werden, welches durch Kristallisation in seine Bestandteile XIa und XIb zerlegt werden kann.

XIa → XIIa und XIb → XIIb

Durch Behandlung der Verbindung der Formel XIa bzw. XIb mit Trifluormethansulfonsäure erhält man L-Phenylalanin-cyclohexylamidtrifluormethansulfonat und die Verbindung der Formel XIIa bzw. XIIb.

XIIa → XIIIa und XIIb → XIIIb

Durch Behandlung der Verbindung der Formel XIIa bzw. XIIb mit Bortribromid in Dichlormethan o.dgl. erhält man die Verbindung der Formel XIIIa bzw. XIIIb.

Ausgehend von der Verbindung der Formel XIIIa sind verschiedene Abwandlungen möglich, welche im nachstehenden Reaktionsschema 3 veranschaulicht werden; dieselben Abwandlungen sind natürlich auch ausgehend von der Verbindung der Formel XIIIb möglich.

## Reaktionsschema 3

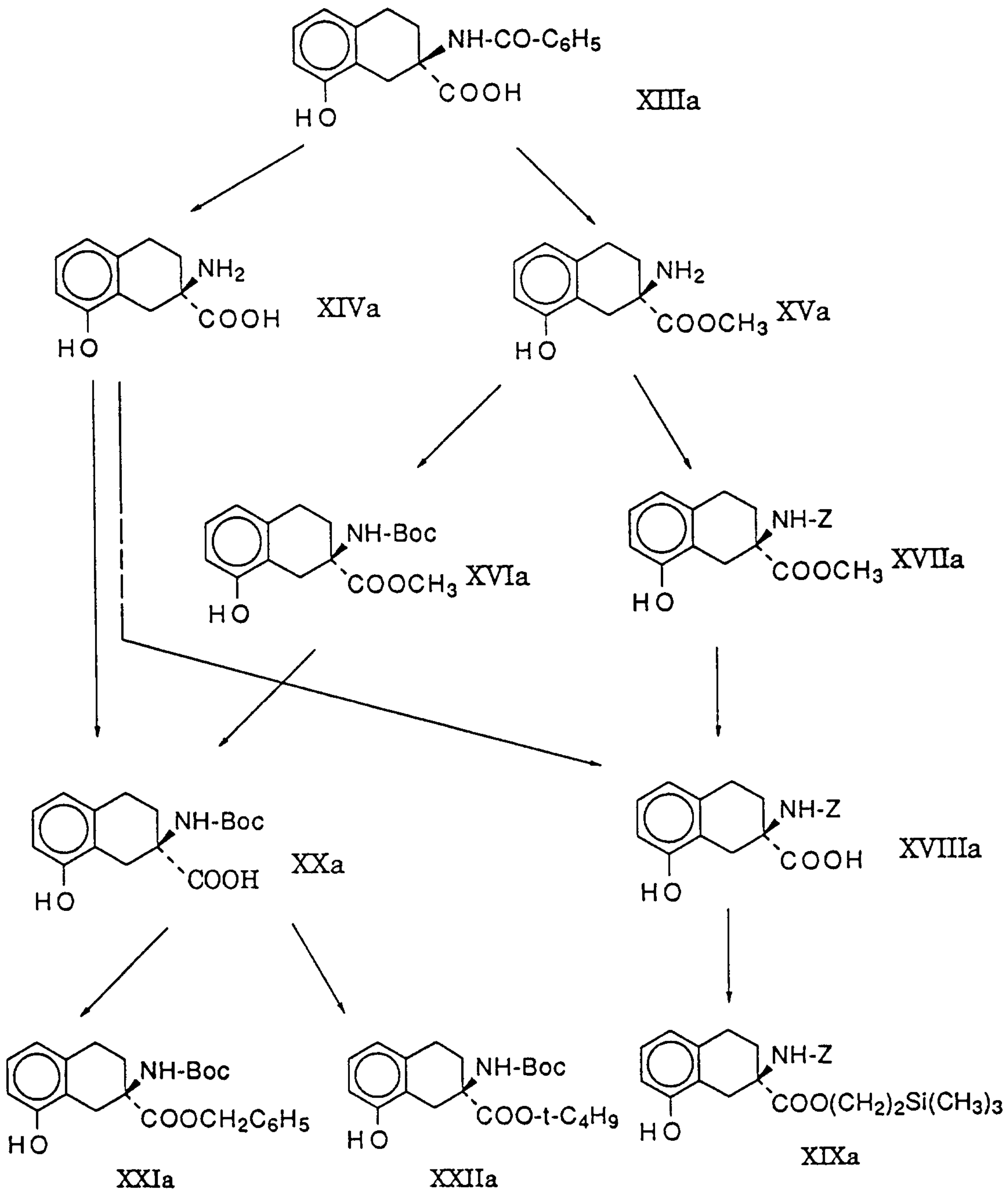

XIIIa

XIVa

XVa

XVIa

XVIIa

XXa

XVIIIa

XXIa

XXIIa

XIXa

Z bedeutet Benzyloxycarbonyl

Boc bedeutet tert.-Butyloxycarbonyl

Die verschiedenen Abwandlungen gemäss Reaktionsschema 3 werden nach an sich bekannten und jedem Fachmann geläufigen Methoden durchgeführt, zweckmässigerweise mittels der nachfolgend erwähnten Reagenzien.

<u>XIIIa → XIVa</u>

25%ige Salzsäure in Dioxan bei 100° (Bombenrohr).

XIIIa → XVa

25%ige Salzsäure in Dioxan bei 100° (Bombenrohr), dann Oxalylchlorid in methanolischer Salzsäure.

XVa → XVIa

Di-tert.-Butyldicarbonat in Dimethylformamid.

XVa → XVIIa

N-(Benzyloxycarbonyloxy)-succinimid und Natriumhydrogencarbonat in Dioxan.

XVIIa → XVIIIa

Lithiumhydroxid in Tetrahydrofuran/Methanol/Wasser.

XIVa → XVIIIa

N-(Benzyloxycarbonyloxy)-succinimid und Natriumhydrogencarbonat in Dioxan.

XVIIIa → XIXa

Trimethylsilyläthanol/N-Aethyl-N'-(3-dimethylaminopropyl)-carbodiimid-hydrochlorid/1-N-Hydroxybenzotriazol/Aethyl-diisopropylamin/N,N-Dimethylaminopyridin in Dimethylformamid.

XIVa → XXa

Di-tert.-butyldicarbonat/Trimethylchlorsilan/Aethyl-diisopropylamin in Dichlormethan.

XVIa → XXa

Lithiumhydroxid in Tetrahydrofuran/Methanol/Wasser.

XXa → XXIa

Benzylbromid/1,8-Diazabicyclo[5.4.0]undec-7-en in Dimethylformamid oder Phenyldiazomethan in Dichlormethan.

XXa → XXIIa

N,N-Dimethylformamid-di-tert.-butylacetal in Toluol.

Verbindungen vom Typus XVIa, XVIIa, XIXa, XXIa und XXIIa können in 5-Stellung bromiert werden, zweckmässigerweise mittels N-Bromsuccinimid in Trifluoräthanol, und anschliessend sind bereits auf dieser Stufe gewisse weitere Variationen (wie weiter unten beschrieben) in 5-Stellung des Tetrahydronaphthalingerüstes möglich (vgl. die verschiedenen Bedeutungsmöglichkeiten von $R^1$ in Formeln Ia und Ib), wobei die phenolische Hydroxylgruppe unter Umständen temporär geschützt werden muss. Weiterhin kann in Verbindungen vom Typus XVIa, XVIIa, XIXa, XXIa und XXIIa oder in entsprechenden 5-Bromverbindungen oder in Abwandlungsprodukten davon mit anderen Substituenten in 5-Stellung die phenolische Hydroxylgruppe durch eine SH-Gruppe ersetzt werden, beispielsweise nach einer der folgenden Methoden:

a) Umsetzung mit N,N-Diäthyl-thiocarbamoylchlorid, gefolgt von Erhitzen und dann alkalischer Hydrolyse, vgl. M.S. Newman, H.A. Karnes, J. Org. Chem. 31, 3980 (1966);

b) Behandlung mit Trifluormethansulfonsäureanhydrid in Pyridin; dann Umsetzung des erhaltenen Trifluormethansulfonats mit Thioharnstoff in Gegenwart eines Nickelkatalysators, gefolgt von Behandlung mit Alkali und dann mit Säure, vgl. K. Takagi, Chem. Lett. 1985, 1307.

Ausgehend von Verbindungen vom Typus XVIa, XVIIa, XIXa, XXIa und XXIIa oder von Abwandlungsprodukten davon, wie vorstehend beschrieben, bzw. von entsprechenden, jedoch aus Verbindung XIIIb hergestellten Verbindungen kann man wie folgt zu Verbindungen der Formeln IVa und IVb gelangen:

Verbindungen der Formel IVa bzw. IVb, worin Y einen Rest der Formel (a) bedeutet, erhält man durch Umsetzung mit entsprechenden o-Nitrohalogenaromaten bzw. entsprechenden, in o-Stellung durch geschütztes Aminomethyl substituierten Halogenaromaten, wie 2-Jodnitrobenzol, 2-Chlor-3-nitropyridin o.dgl. bzw. Benzyl N-2-brombenzylcarbamat o.dgl., und anschliessende Reduktion der Nitrogruppe bzw. Abspaltung der Schutzgruppe.

Verbindungen der Formel IVa bzw. IVb, worin Y einen Rest der Formel (b) bedeutet, erhält man durch Umsetzung mit einem entsprechenden, in 3-Stellung durch geschütztes Hydroxy substituierten Alkylhalogenid, wie 1-Brom-3-tert.butyldimethylsilyloxy-2-methylpropan, Abspaltung der O-Schutzgruppe, Ersatz der Hydroxygruppe durch eine Abgangsgruppe, wie Tosyloxy, Ersatz der Abgangsgruppe durch eine Azidgruppe, z.B. mittels Natriumazid, und Reduktion der Azidgruppe zur Aminogruppe, zweckmässigerweise durch katalytische Hydrierung, z.B. in Gegenwart eines Palladiumkatalysators, wie Pd-C. Bei besagter Hydrierung kann, falls als Lösungsmittel ein niederes Alkanol, wie Ethanol, verwendet wird, unter Umständen neben der Aminoverbindung auch die entsprechende nied.-Alkylaminoverbindung entstehen (also z.B. die entsprechende Ethylaminoverbindung); die Auftrennung eines solchen Gemisches kann auf dieser oder auch auf einer späteren Stufe erfolgen.

In den erhaltenen Verbindungen der Formeln IVa und IVb sind verschiedene Abwandlungen möglich, wobei die Aminogruppe notwendigenfalls vorgängig geschützt und anschliessend wieder freigesetzt wird.

Einerseits können Variationen bezüglich der Schutzgruppen durchgeführt werden. So kann z.B. eine tert.-Butoxycarbonylaminogruppe in eine Fluorenylmethoxycarbonylaminogruppe, ein tert.-Butylester in einen Pentafluorphenylester oder ein Benzylester in einen Pentafluorphenylester übergeführt werden, was nach allgemein üblichen und jedem Fachmann geläufigen Methoden erfolgen kann.

Anderseits werden Variationen bezüglich der 5-Stellung des Tetrahydronaphthalingerüstes zweckmässigerweise auf dieser Stufe durchgeführt; die Einführung von Brom erfolgt jedoch zweckmässigerweise bereits auf einer früheren Stufe, nämlich auf der Stufe der Verbindungen von Typus XVIa, XVIIa, XIXa, XXIa und XXIIa. So können Verbindungen der Formel IVa und IVb, worin $R^1$ Brom bedeutet, beispielsweise wie folgt abgewandelt werden:

a) Umsetzung mit Organozinnverbindungen in Gegenwart eines Palladiumkatalysators, vgl. T.N. Mitchell, Synthesis 1992, 803-815; J.K. Stille, Angew. Chem. 98, 504-519 (1986); D.R. McKean, G. Parinello, A.F. Renaldo, J.K. Stille, J. Org. Chem. 52, 422 (1987). Beispielsweise erhält man mittels 3,4-Dimethoxyphenyl-trimethylstannan in Gegenwart von Tetrakis-(triphenylphosphin)-palladium in Dioxan eine Verbindung der Formel IVa bzw. IVb, worin $R^1$ 3,4-Dimethoxyphenyl bedeutet.

b) Umsetzung mit Boronaten in Gegenwart eines Palladiumkatalysators, vgl. X. Wang, V. Sniekus, Tetrahedron Lett. 1991, 4879; B.I. Alo, A. Kandil, P.A. Patil, M.J. Sharp, M.A. Siddiqui, V. Snieders, J. Org. Chem. 56, 3763 (1991); T.Oh-e, N. Miyaura, A. Suzuki, Synlett 1990, 221.

Methoden a) und b) eignen sich zur Einführung von Aryl-, Alkenyl-, Alkinyl- und Alkyl-Resten.

c) Umsetzung mit Kohlenmonoxid und einem Alkohol in Gegenwart von Palladium-diacetat o.dgl. und einer Base, vgl. J.K Stille, P.K. Wong, J. Org. Chem. 40, 532 (1975); A. Cowell, J.K. Stille, J. Amer. Chem. Soc. 102, 4193 (1980). Diese Methode eignet sich zur Einführung von Alkoxycarbonylgruppen.

d) Umsetzung mit Kohlenmonoxid und Wasserstoff in Gegenwart von Triäthylamin o.dgl. und Bis-(triphenylphosphin)Palladiumdichlorid o.dgl., vgl. A. Schoenberg, R.F. Heck, J. Amer. Chem. Soc. 96, 7761 (1974); H. Yoshida, N. Sugita, K. Kudo, Y. Takezaki, Bull. Chem. Soc. Japan 49, 1681 (1976). Diese Methode eignet sich zur Einführung der Formylgruppe.

e) Umsetzung mit Kaliumcyanid in Gegenwart von 1,1'-Bis-(diphenylphosphino)-ferrocen und Bis-(dibenzylidenaceton)-di-palladium in N,N-Dimethylacetamid, vgl. K. Takagi, Y. Sakakibara, Chem. Lett. 1989, 1957. Diese Methode eignet sich zur Einführung der Cyanogruppe.

f) Umwandlung der Formylgruppe in eine Alkenylgruppe durch Wittig-Reaktion.

g) Behandlung mit tert.-Butyllithium und anschliessende Umsetzung mit geeigneten Elektrophilen wie z.B. N-Formylpiperidin (liefert Formyl), Chlorameisensäureester (liefert Alkoxycarbonyl) usw.

h) Ullmann-Kopplung mit einem Phenol in Gegenwart von Natriumhydrid und Kupferbromid-Dimethylsulfid-Komplex in Pyridin, vgl. D.L. Boger, D. Yohannes, J. Org. Chem. 55, 6000 (1990); D.A. Evans, J.A. Ellmann, J. Amer. Chem. Soc. 111, 1063 (1989). Diese Methode eignet sich zur Einführung von Aryloxygruppen.

i) Umsetzung Alkoholen, insbesondere primären Alkoholen, unter Phasentransfer-Katalyse (z.B. PEG-6000, KOH), vgl. R. Neumann, Y. Sasson, Tetrahedron 39, 3437 (1983). Diese Methode eignet sich zur Einführung von Alkoxy- und Aralkoxygruppen.

k) Umwandlung der Formylgruppe in die Hydroxygruppe mittels modifizierter Bayer-Villiger-Reaktion (Natriumpercarbonat/Trifluoressigsäure), vgl. G. Olah, Synthesis 1991, 739.

Ausgehend von Verbindungen der Formel IVa bzw. IVb kann man zu Verbindungen der Formel IIa bzw. IIb gelangen, indem man zunächst mit einer den Rest $A^1$ bzw. $A^3$ liefernden Aminosäurekomponente und hierauf mit einer den Rest $A^2$ bzw. $A^4$ liefernden Aminosäurekomponente kuppelt, anschliessend die durch $Z^1$ geschützte Carboxylgruppe freisetzt, dann cyclisiert und schliesslich die durch $Z^2$ geschützte Aminogruppe freisetzt.

Zu Verbindungen der Formel IIIa bzw. IIIb kann man ausgehend von Verbindungen der Formel IVa bzw. IVb gelangen, indem man zunächst die durch $Z^2$ geschützte Aminogruppe freisetzt, diese mit einer den Rest $R^2$ liefernden Peptidkomponente kuppelt und dann mit einer den Rest $A^1$ bzw. $A^3$ und hierauf mit einer den Rest $A^2$ bzw. $A^4$ liefernden Aminosäurekomponente kuppelt.

Die Herstellung der Verbindungen der Formel IIa bzw. IIb und der Formel IIIa bzw. IIIb aus den Verbindungen der Formel IVa bzw. IVb erfolgt nach in der Peptidchemie üblichen und jedem Fachmann geläufigen Methoden.

Wird im Zuge dieser Synthese als den Rest $A^1$ bzw. $A^3$ liefernde Aminosäurekomponente ein Asparaginsäurederivat eingesetzt, so kann in der erhaltenen Verbindung die Carboxylgruppe des Aspartylrests nicht in freier Form, sondern als Glied eines mit dem Stickstoffatom der $A^1$ bzw. $A^3$ mit $A^2$ bzw. $A^4$ verbindenden Amidbindung gebildeten fünfgliedrigen Rings vorliegen; dieser Ring kann - zweckmässigerweise erst nach der Ueberführung in eine Verbindung der Formel Ia bzw. Ib - wieder geöffnet werden, z.B. mittels Lithiumhydroxid o.dgl.

Die Verbindungen der Formeln Ia und Ib enthalten Aminosäuresequenzen (vgl. den Rest $R^2$), wobei in den Verbindungen der Formel Ia die Konformationen von rechtsgängigen peptidischen α-Helices (mit L-Aminosäureresten) stabilisiert und die Konformationen von linksgängigen peptidischen α-Helices (mit D-Aminosäureresten) destabilisiert werden, wogegen in den Verbindungen der Formel Ib die Konformationen von linksgängigen peptidischen α-Helices (mit D-Aminosäureresten) stabilisiert und die Konformationen von rechtsgängigen peptidischen α-Helices (mit L-Aminosäureresten) destabilisiert werden.

Die den Verbindungen der Formel IIa bzw. IIb entsprechenden Strukturelemente in den Verbindungen der Formel Ia bzw. Ib können als Mimetica einer α-helicalen Windung aufgefasst werden. Die Stabilisierung bzw. Fortpflanzung der Helizität in Verbindungen der Formel Ia bzw. Ib beruht darauf, dass sich einerseits das für α-Helices charakteristische i→i+4 Wasserstoffbrücken-Netzwerk [vgl. L. Pauling, R.B. Corey, H.R. Branson, Proc. Natl. Acad. Sci. 37, 205 (1951)] ausbilden kann und dass andererseits der 2-Amino-1,2,3,4-tetrahydro-naphthalin-2-carbonsäure-Baustein am asymmetrischen C-Atom in 2-Stellung inhaerent α-helicale Konformationen induziert, vgl. C. Spiegler, Synthesis and conformational studies of peptides containing novel α,α-disubstituted amino acids, Dissertation Universität Zürich, 1993).

Wie aus Figur 1 hervorgeht, beträgt die in Trifluoraethanol/Wasser (1:1) ermittelte Helizität des Endprodukts von Beispiel 5.1.3.a in neutralem Medium 81% und in saurem Medium (HCl) 92%, diejenige eines Referenz-Peptids gleicher Länge jedoch in neutralem und saurem Medium 50% [die Berechnung der Helizität wurde analog zu Y.-H. Chen, J.T. Wang, H.H. Martinez, Biochemistry 11, 4120 (1972) und Y.-H. Chen, J.T. Wang, K.H. Chau, Biochemistry 13, 3330 (1974) durchgeführt].

Die Priorität der Aminosäurereste $A^1$ und $A^2$ bzw. $A^3$ und $A^4$ in den Verbindungen der Formel Ia bzw. Ib ist etwa analog zur Kompatibilität von L- und D-Aminosäuren in peptidischen α-Helices, vgl. A. Horovitz, J.M. Matthews, A.R. Fersht, J. Mol. Biol. 227, 560-568 (1992).

Aufgrund ihrer Eigenschaften eignen sich Verbindungen der Formel Ia bzw. Ib als Mimetica von exponierten helicalen Domänen von Proteinen, um deren Rolle bezüglich der Wechselwirkungen mit anderen Proteinen (Rezeptoren, Enzyme o.dgl.) oder mit DNA bzw. RNA aufzuklären. Insbesondere können so mittels Verbindungen der Formel Ia bzw. Ib Aminosäuresequenzen mit biologischer Aktivität ermittelt werden. Die Verbindungen der Formel Ia bzw. Ib eignen sich deshalb als Forschungshilfsmittel ("Research Tools"), um biologisch aktive Peptidsequenzen bereitzustellen. Die Verbindungen der Formel Ia bzw. Ib sind aber auch potentiell als Arzneimittel geeignet, wobei der jeweilige therapeutische Verwendungszweck in erster Linie von der Natur, der Anzahl und der Reihenfolge der im Molekül vorhandenen Aminosäurereste abhängt.

In den nachfolgenden Beispielen, welche die Erfindung näher erläutern, ihren Umfang aber in keiner Weise einschränken sollen, sind sämtliche Temperaturen in Celsiusgraden angegeben. Die Bezeichnung der Beispiele, deren Endprodukte am asymmetrischen C-Atom in 2-Stellung des 2-Amino-1,2,3,4-tetrahydro-naphthalin-2-carbonsäure-Bausteins die R-Konfiguration aufweisen, endet stets mit "a", diejenige der Beispiele, deren Endprodukte am fraglichen C-Atom die S-Konfiguration aufweisen, endet durchwegs mit "b".

Beispiel 1.1

Zu einer Lösung von 120.0 g (750 mMol) 1,7-Dihydroxy-naphthalin in einem Gemisch von 300 ml Tetrahydrofuran, 142 ml tert.-Butylalkohol und 1000 ml flüssigem Ammoniak wurden unter Argon und Kühlen (Trockeneis/Aceton) analog zu D.W. Johnson, L.N. Mander, Aust. J. Chem. 1974, 27, 1277 innerhalb von 20 Minuten 15,0 g Lithiummetallstücke zugegeben. Das Gemisch wurde 1 Stunde gerührt und dann mit 400 ml Methanol versetzt, worauf 1 Stunde bei -70° weitergerührt, der Ammoniak unter $N_2$-Strom entfernt, der Rückstand mit wässriger 10%iger HCl angesäuert und mit 3 x 2.0 l Essigsäureäthylester extrahiert wurde. Die organische Phase wurde zweimal mit 1,0 l gesättigter Kochsalzlösung gewaschen, über $MgSO_4$ getrocknet und eingedampft. Der Rückstand wurde in 500 ml Essigsäureäthylester und 40 ml Aethanol gelöst, worauf mit 1,0 l 38-40%iger Natriumhydrogensulfitlösung versetzt und das Gemisch während 18 Stunden geschüttelt wurde. Der Niederschlag wurde abfiltriert, mit verdünnter $NaHSO_3$-Lösung und Essigester gewaschen und am Vakuum über Sicapent getrocknet, wobei 96.2 g (48%) (rac)-2,8-Dihydroxy-1,2,3,4-tetrahydronaphthalin-2-sulfonsäure-Natriumsalz erhalten wurden; Smp. 160-162°.

Beispiel 1.2

Eine Suspension von 48.0 g (180 mMol) (rac)-2,8-Dihydroxy-1,2,3,4-tetrahydro-naphthalin-2-sulfonsäure-Natriumsalz, 20.2 g (310 mMol) Kaliumcyanid und 118.1 g (1 Mol) Ammoniumcarbonat in 800 ml Aethanol/Wasser (4:1) wurde während 2 Stunden bei 65° Innentemperatur gerührt, dann abgekühlt und auf ein Gemisch von Eis und 1.2 l 2N wässriger HCl gegossen. Die Suspension wurde über Nacht stehengelassen und dann filtriert. Der Rückstand wurde mit $H_2O$ gewaschen und am Vakuum über Sicapent getrocknet, wobei 31.5 g (75.4%) (rac)-3',4'-Dihydroxy-8'-hydroxyspiro[imidazolidin-4,2'(1'H)-naphthalin]-2,5-dion erhalten wurden; Smp. 232-234° (Zers.).

Beispiel 1.3

Eine Suspension von 30.0 g (129 mMol) (rac)-3',4'-Dihydroxy-8'-hydroxyspiro[imidazolidin-4',2'(1'H)-naphthalin]-2,5-dion und 203.8 g $Ba(OH)_2 \cdot 8H_2O$ in 800 ml Wasser wurde im Stahlautoklaven während 24 Stunden bei 125° gerührt, dann abgekühlt und mit 4N $H_2SO_4$-Lösung angesäuert, worauf während 1 Stunde auf dem Wasserbad erhitzt, abgekühlt, filtriert und der Filterrückstand mit verdünnter $H_2SO_4$-Lösung gewaschen wurde. Das saure Filtrat wurde bis auf ein Volumen von ca. 300 ml eingeengt und mit konzentrierter wässriger Ammoniaklösung neutralisiert, worauf ein Niederschlag ausfiel. Die Suspension wurde über Nacht stehengelassen und dann filtriert. Der Rückstand wurde am Vakuum über Sicapent getrocknet, wobei 20.5 g (83.1%) (rac)-2-Amino-8-hydroxy-1,2,3,4-tetrahydro-naphthalin-2-carbonsäure erhalten wurden; Smp. >280° (Zers.).

Beispiel 1.4

Zu einer Lösung von 19.0 g (99.4 mMol) (rac)-2-Amino-8-hydroxy-1,2,3,4-tetrahydro-naphthalin-2-carbonsäure in 210 ml 1N NaOH wurden unter Eiskühlung und Rühren gleichzeitig aus 2 Tropftrichtern 50 ml 2N NaOH und 40,3 ml (345 mMol) Benzoylchlorid so zugetropft, dass die Innentemperatur nicht über 15° anstieg. Das Reaktionsgemisch wurde anschliessend langsam auf Raumtemperatur gebracht, 2 Stunden gerührt, mit 2N HCl angesäuert und zweimal mit 250 ml Essigester extrahiert. Die vereinigten organischen Phasen wurden über $MgSO_4$ getrocknet und eingedampft. Der Rückstand wurde in 300 ml Dioxan gelöst, und die Lösung wurde unter Eiskühlung mit 150 ml 2N NaOH versetzt. Das Reaktionsgemisch wurde über Nacht gerührt, mit 4N HCl angesäuert und dreimal mit 300 ml Essigester extrahiert. Die vereinigten organischen Phasen wurden über $MgSO_4$ getrocknet und eingedampft. Der Rückstand wurde in 500 ml Diäthyläther/Hexan (4:1) aufgenommen, und die Suspension wurde über Nacht gerührt und dann filtriert. Der Filterrückstand wurde am Hochvakuum getrocknet, wobei 28.6 g (92.4%) (rac)-2-Benzamido-8-hydroxy-1,2,3,4-tetrahydro-naphthalin-2-carbonsäure erhalten wurden. Smp. 234-236° (Zers.).

Beispiel 1.5

Zu einer Suspension von 18.0 g (57.8 mMol) (rac)-2-Benzamido-8-hydroxy-1,2,3,4-tetrahydro-naphthalin-2-carbonsäure in 150 ml Dichlormethan wurden unter Kühlen portionenweise 12.53 g (60.7 mMol) N,N-Dicyclohexylcarbodiimid zugegeben. Das Gemisch wurde während 2 Stunden bei Raumtemperatur gerührt und dann filtriert, worauf der Filterrückstand mit Dichlormethan gewaschen wurde. Das Filtrat wurde eingedampft, und der Rückstand wurde am Hochvakuum getrocknet und in 150 ml absolutem Dioxan gelöst. Die Lösung wurde unter Eiskühlung und Argon portionenweise mit 2.80 g Natriumhydrid-Suspension (55%) versetzt, worauf 30 Minuten bei Raumtemperatur gerührt und dann 16.5 ml Dimethylsulfat zugegeben wurden. Die Suspension wurde 1 Stunde bei 80° gerührt, abgekühlt und dann auf eine Mischung von Eis, 10%iger Natriumhydrogenphosphat-Lösung und Essigester gegossen. Die wässrige Phase wurde mit Essigester extrahiert, und die vereinigten organischen Phasen wurden über $MgSO_4$ getrocknet und einge-

engt. Der Rückstand wurde an 500 g Kieselgel mit Essigester/Hexan (1:4) chromatographiert, wobei nach Kristallisation aus Diäthyläther/Hexan (1:2) und Trocknen am Hochvakuum 13.6 g (76.5%) (rac)-3,4-Dihydro-8-methoxy-2'-phenyl-spiro-[naphthalin-2(1H),4'(5'H)-oxazol]-5'-on erhalten wurden. Smp. 108-109°.

Beispiel 1.6

Ein Gemisch von 12.6 g (41 mMol) (rac)-3,4-Dihydro-8-methoxy-2'-phenyl-spiro[naphthalin-2(1H),4'(5'H)-oxazol]-5'-on und 15.15 g (61.5 mMol) L-phenylalanin-cyclohexylamid in 120 ml N-Methylpyrrolidon wurde während 18 Stunden bei 60° gerührt, dann abgekühlt und anschliessend auf ein Gemisch von 300 ml Wasser und 500 ml Essigester gegossen. Die organische Phase wurde zweimal mit 250 ml 0.5N HCl extrahiert, und die vereinigten wässrigen Phasen wurden mit Essigester extrahiert. Die vereinigten organischen Phasen wurden mit gesättigter Kochsalzlösung gewaschen, über $MgSO_4$ getrocknet und eingedampft. Der Rückstand wurde während 2 Stunden in 300 ml Essigester suspendiert, worauf die Suspension filtriert und der Rückstand mit Essigester gewaschen, aus Essigester/Hexan (6:1) umkristallisiert und getrocknet wurde. Man erhielt 10.44 g (46%) $N^2$-[(S)-2-Benzamido-8-methoxy-1,2,3,4-tetrahydro-naphthalin-2-carbonyl]-L-phenylalanin-cyclohexylamid. Smp. 186-188°. $[\alpha]_D$ = +12.0° (c=0.2, Methanol).

Das Filtrat wurde eingeengt, und der Rückstand wurde an 1 kg Kieselgel mit Diäthyläther/Isopropanol (99.5:0.5) chromatographiert, worauf nach Umkristallisation aus Essigester/Hexan (1:1) und Trocknen am Hochvakuum 10.25 g (45.2%) $N^2$-[(R)-2-Benzamido-8-methoxy-1,2,3,4-tetrahydro-naphthalin-2-carbonyl]-L-phenylalanin-cyclohexylamid erhalten wurden. Smp. 191-192°. $[\alpha]_D$ = -21.0° (c=0.2, Methanol).

Beispiel 1.7.b

Zu einer Suspension von 7.5 g (13.54 mMol) $N^2$-[(S)-2-Benzamido-8-methoxy-1,2,3,4-tetrahydro-naphthalin-2-carbonyl]-L-phenylalanin-cyclohexylamid in 35 ml Methanol wurden unter Argon und Eiskühlung 7.1 ml (80.74 mMol) Trifluormethansulfonsäure zugetropft. Das Gemisch wurde im Bombenrohr während 4 Stunden bei 80° gerührt, dann abgekühlt und anschliessend auf ein Volumen von ~20 ml eingeengt. Unter Rühren wurden 50 ml Dichlormethan zugegeben, die Suspension wurde filtriert, und der Filterrückstand wurde mit Dichlormethan gewaschen und getrocknet, wobei 4.48 g (83%) L-Phenylalanin-cyclohexylamid-trifluormethansulfonat isoliert wurden. Das Filtrat wurde mit Wasser gewaschen, über $MgSO_4$ getrocknet und eingeengt. Der Rückstand wurde an 350 g Kieselgel mit Essigester/Hexan (2:3) chromatographiert, worauf 4.0 g (87%) (S)-2-Benzamido-8-methoxy-1,2,3,4-tetrahydro-naphthalin-2-carbonsäuremethylester erhalten wurden. $[\alpha]_D$ = +144,0° ($CHCl_3$, c=0.2). IR(KBr): 3366w(br.), 3062w, 2998w, 2948w, 1739s, 1647s, 1586m, 1527s, 1468s, 1437m, 1292m, 1259s, 1099m, 1047m, 774w, 713m. (D. Obrecht, Helv. Chim. Acta 1992, 75, 1666).

Beispiel 1.7.a

In Analogie zu Beispiel 1.7.b wurden aus 8.79 g (15.87 mMol) $N^2$-[(R)-2-Benzamido-8-methoxy-1,2,3,4-tetrahydro-naphthalin-2-carbonyl]-L-phenyl alanin-cyclohexylamid 4.78 g (88%) (R)-2-Benzamido-8-methoxy-1,2,3,4-tetrahydro-naphthalin-2-carbonsäure-methylester erhalten. $[\alpha]_D$ = -142.5° (c=0.2, $CHCl_3$).

Beispiel 1.8.b

Zu einer Lösung von 1.95 g (5.75 mMol) (S)-2-Benzamido-8-methoxy-1,2,3,4-tetrahydro-naphthalin-2-carbonsäure-methylester in 15 ml Dichlormethan wurden unter Argon und Eiskühlung 28.7 ml Bortribromid-Lösung (1M in Dichlormethan) zugetropft. Das Reaktionsgemisch wurde 30 Minuten bei 0° und 3 Stunden bei Raumtemperatur gerührt und dann auf eine Mischung von Eis, gesättigter Ammoniumchlorid-Lösung und Essigester gegossen. Die organische Phase wurde abgetrennt, mit gesättigter Kochsalzlösung extrahiert, über $MgSO_4$ getrocknet und eingeengt. Der Rückstand wurde am Hochvakuum getrocknet, in Diäthyläther/Hexan (1:4) suspendiert und abfiltriert. Der Filterrückstand wurde getrocknet, wobei 1.75 g (97.8%) (S)-2-Benzamido-8-hydroxy-1,2,3,4-tetrahydro-naphthalin-2-carbonsäure erhalten wurden. $[\alpha]_D$ = +84.3° (c=0.3, Methanol). IR(KBr): 3362m(br.), 3064w, 3022w, 2976w, 2938w, 2620w, 1718s, 1644s, 1588m, 1523s, 1487m, 1467s, 1330m, 1278s, 1082w, 716m.

Beispiel 1.8.a

In Analogie zu Beispiel 1.8.b wurden aus 2.71 g (7.98 mMol) (R)-2-Benzamido-8-methoxy-1,2,3,4-tetrahydro-naphthalin-2-carbonsäure-methylester 2.42 g (97.4%) (R)-2-Benzamido-8-hydroxy-1,2,3,4-tetrahydro-naphthalin-2-carbonsäure erhalten. $[\alpha]_D$ = -85.7° (c=0.3, Methanol).

Beispiel 2.1.b

Eine Lösung von 1.75 g (5.62 mMol) (S)-2-Benzamido-8-hydroxy-1,2,3,4-tetrahydro-naphthalin-2-carbonsäure in 12 ml 25%iger Salzsäure und 6 ml Dioxan wurde im Bombenrohr 8 Stunden auf 110° erhitzt, dann abgekühlt und anschliessend zur Trockene eingedampft. Der Rückstand wurde über Nacht am Hochvakuum über Sicapent getrocknet und dann unter Rühren in 20 ml Diäthyläther suspendiert, worauf die Suspension filtriert wurde. Der Filterrückstand wurde mit Diäthyläther gewaschen und am Hochvakuum getrocknet und dann in 3 ml Methanol und 3 ml 15%iger methanolischer Salzsäure gelöst. Die Lösung wurde unter Argon und Eiskühlung mit 0.42 ml (3.89 mMol) Oxalylchlorid versetzt, worauf das Gemisch im Bombenrohr 20 Stunden bei 50° gerührt, dann abgekühlt und anschliessend auf eine Mischung von gesättigter Natriumhydrogencarbonat-Lösung und Chloroform gegossen wurde. Die wässrige Phase wurde mit Chloroform/Methanol (6:1) extrahiert. Die vereinigten organischen Phasen wurden über $MgSO_4$ getrocknet, filtriert und eingeengt. Der Rückstand wurde an 150 g Kieselgel mit Chloroform/Methanol (6:1) chromatographiert, wobei nach Umkristallisation aus Essigester/Hexan 1.05 g (84.4%) (S)-2-Amino-8-hydroxy-1,2,3,4-tetrahydro-naphthalin-2-carbonsäure-methylester erhalten wurden. Smp. 183-185°. $[\alpha]_D$ = -22.7° (c=0.15, Methanol).

Beispiel 2.1.a

In Analogie zu Beispiel 2.1.b wurden aus 2.40 g (7.71 mMol) (R)-2-Benzamido-8-hydroxy-1,2,3,4-tetrahydro-naphthalin-2-carbonsäure 1.60 g (95.3%) (R)-2-Amino-8-hydroxy-1,2,3,4-tetrahydro-naphthalin-2-carbonsäuremethylester erhalten. Smp. 184-185°.

Beispiel 2.2.b

Eine Suspension von 9.2 g (29.55 mMol) (S)-2-Benzamido-8-hydroxy-1,2,3,4-tetrahydro-naphthalin-2-carbonsäure in 45 ml Dioxan und 62 ml 25%iger Salzsäure wurde im Bombenrohr 24 Stunden auf 100° erhitzt, dann abgekühlt, auf die Hälfte des Volumens eingeengt und mit Essigester extrahiert. Die organische Phase wurde mit Wasser gewaschen, worauf die vereinigten wässrigen Phasen auf ein Volumen von 200 ml reduziert und mit konzentrierter Ammoniaklösung auf pH 7 gestellt wurden. Die erhaltene Lösung wurde über MCI-Gel (CHP20P; 75-150 µ) filtriert (Wasser, Wasser/Methanol (95:5) → Wasser/Methanol (9:1)), worauf nach Trocknen über Sicapent 5.30 g (86.6%) (S)-2-Amino-8-hydroxy-1,2,3,4-tetrahydro-naphthalin-2-carbonsäure erhalten wurden. Smp. >270° (Zers.). $[\alpha]_D$ = +7.0° (c=0.2, 0,1N HCl).

Beispiel 2.2.a

In Analogie zu Beispiel 2.2.b wurden aus 4.50 g (14.45 mMol) (R)-2-Benzamido-8-hydroxy-1,2,3,4-tetrahydro-naphthalin-2-carbonsäure 2.49 g (83%) (R)-2-Amino-8-hydroxy-1,2,3,4-tetrahydro-naphthalin-2-carbonsäure erhalten. Smp. >272° (Zers.). $[\alpha]_D$ = -5.0° (c=0.2, 0,1N HCl).

Beispiel 2.3.b

Zu einer Lösung von 780 mg (3.53 mMol) (S)-2-Amino-8-hydroxy-1,2,3,4-tetrahydro-naphthalin-2-carbonsäure-methylester in 10 ml DMF wurde unter Eiskühlung und Argon eine Lösung von 924 mg (4.24 mMol) Di-tert.-Butyldicarbonat in 1.5 ml DMF zugegeben. Das Reaktionsgemisch wurde 30 Minuten bei 0° und 18 Stunden bei Raumtemperatur gerührt und dann auf eine Mischung von Wasser und Essigester gegossen. Die organische Phase wurde abgetrennt, mit Wasser extrahiert, über $MgSO_4$ getrocknet und eingeengt. Der Rückstand wurde an 100 g Kieselgel mit Essigester/Hexan (1:2) chromatographiert, worauf nach Umkristallisation aus Diäthyläther/Hexan und Trocknen 1.0 g (88.1%) (S)-2-(tert.Butoxycarbonylamino)-8-hydroxy-1,2,3,4-tetrahydro-naphthalin-2-carbonsäure-methylester erhalten wurden. Smp. 186-187°. $[\alpha]_D$ = +95.0° (c=0.2, Chloroform).

Beispiel 2.3.a

In Analogie zu Beispiel 2.3.b wurden aus 1.46 g (6.60 mMol) (R)-2-Amino-8-hydroxy-1,2,3,4-tetrahydro-naphthalin-2-carbonsäure-methylester 1.80 g (85%) (R)-2-(tert.Butoxycarbonylamino)-8-hydroxy-1,2,3,4-tetrahydro-naphthalin-2-carbonsäure-methylester erhalten. Smp. 186-187°. $[\alpha]_D$ = -93.5° (c=0.2, Chloroform).

Beispiel 2.4.b

Eine Suspension von 1.1 g (4.27 mMol) (S)-2-Amino-8-hydroxy-1,2,3,4-tetrahydro-naphthalin-2-carbonsäure-methylester in 22 ml Dioxan wurde bei Raumtemperatur mit 2.12 g (8.54 mMol) N-(Benzyloxycarbonyloxy)succinimid

und 717 mg (8.54 mMol) Natriumhydrogencarbonat versetzt. Das Reaktionsgemisch wurde 4 Stunden bei Raumtemperatur gerührt und dann auf eine Mischung von gesättigter Natriumchlorid-Lösung und Essigester gegossen. Die organische Phase wurde abgetrennt, über $MgSO_4$ getrocknet und eingeengt. Der Rückstand wurde an 200 g Kieselgel mit Chloroform/Methanol (98:2→95:5) chromatographiert, worauf nach Trocknen 1.30 g (86.9%) (S)-2-Benzyloxycarbonylamino-8-hydroxy-1,2,3,4-tetrahydro-naphthalin-2-carbonsäure-methylester erhalten wurden. Smp. 58-68° (Sintern). $[\alpha]_D$ = +85.0° (c=0.2, Chloroform).

Beispiel 2.5.b

Eine Lösung von 800 mg (2.26 mMol) (S)-2-Benzyloxycarbonylamino-8-hydroxy-1,2,3,4-tetrahydro-naphthalin-2-carbonsäure-methylester in 10 ml THF/Methanol/Wasser (3:1:1) wurde unter Eiskühlung mit 695 mg Lithiumhydroxid • 1 $H_2O$ versetzt. Das Reaktionsgemisch wurde 1 Stunde bei 0° und 3 Stunden bei Raumtemperatur gerührt und dann auf eine Mischung von Eis, 0.5N Salzsäurelösung und Chloroform gegossen. Die Wasserphase wurde abgetrennt und mit Choroform erschöpfend extrahiert. Die vereinigten organischen Fraktionen wurden über Natriumsulfat getrocknet und eingeengt. Nach Trocknen des Rückstands erhielt man 555 mg (72%) (S)-2-Benzyloxycarbonyl-8-hydroxy-1,2,3,4-tetrahydro-naphthalin-2-carbonsäure als beiges Pulver. Smp. 68-75° (Sintern). $[\alpha]_D$ = +83.5° (c= 0.2, Chloroform).

Beispiel 2.6.b

Zu einer Suspension von 100 mg (0.48 mMol) (S)-2-Amino-8-hydroxy-1,2,3,4-tetrahydro-naphthalin-2-carbonsäure in 2 ml Dioxan/Wasser (1:1) wurden 143 mg (0.576 mMol) N-(Benzyloxycarbonyl)-succinimid und 80.6 mg (0.96 mMol) Natriumhydrogencarbonat zugegeben. Das Reaktionsgemisch wurde über Nacht bei Raumtemperatur gerührt, danach nochmals mit 40 mg (0.336 mMol) Natriumhydrogencarbonat und 83.6 mg (0.48 mMol) N-(Benzyloxycarbonyl)-succinimid versetzt, 4 Stunden bei Raumtemperatur gerührt und dann auf eine Mischung von gesättigter Natriumchlorid-Lösung und Essigester gegossen. Die organische Phase wurde abgetrennt, über $MgSO_4$ getrocknet und eingeengt. Der Rückstand wurde an 20 g Kieselgel mit Chloroform/Methanol/Wasser (6:3:0.5) chromatographiert, worauf nach Trocknen 66 mg (38.7%) (S)-2-Benzyloxycarbonylamino-8-hydroxy-1,2,3,4-tetrahydro-naphthalin-2-carbonsäure als beiges Pulver erhalten wurden. Smp. 65-75° (Sintern).

Beispiel 2.7.b

Eine Lösung von 620 mg (1.82 mMol) (S)-Benzyloxycarbonylamino-8-hydroxy-1,2,3,4-tetrahydro-naphthalin-2-carbonsäure in 6 ml N,N-Dimethylformamid wurde bei 0° mit 521 mg (2.73 mMol) N-Aethyl-N'-(3-dimethylaminopropyl)-carbodiimid-hydrochlorid, 489 mg (3.64 mMol) 1-N-Hydroxylbenzotriazol, 502 µl (2.91 mMol) Aethyldiisopropylamin und 627 µl (4.37 mMol) Trimethylsilyläthanol versetzt. Das Reaktionsgemisch wurde 1 Stunde bei 0° gerührt, dann mit 50 mg N,N-Dimethylaminopyridin und weiteren 627 µl (4.37 mMol) Trimethylsilyläthanol versetzt, hierauf 24 Stunden bei Raumtemperatur gerührt und anschliessend auf eine Mischung von Wasser und Dichlormethan gegossen. Die wässrige Phase wurde mit 0.1N Salzsäure angesäuert und mit Dichlormethan erschöpfend extrahiert. Die vereinigten organischen Phasen wurden über $MgSO_4$ getrocknet und eingeengt. Der Rückstand wurde an 100 g Kieselgel mit Hexan/Essigester (4:1→1:1) chromatographiert, worauf nach Trocknen 449 mg (56%) (S)-2-Benzyloxycarbonylamino-8-hydroxy-1,2,3,4-tetrahydro-naphthalin-2-carbonsäure-trimethylsilyläthylester erhalten wurden. Smp. 141-144°. $[\alpha]_D$ = +68.0° (c=0.2, Chloroform).

Beispiel 2.8.b

Zu einer Suspension von 513 mg (2.68 mMol) (S)-2-Amino-8-hydroxy-1,2,3,4-tetrahydro-naphthalin-2-carbonsäure in 10 ml Dichlormethan wurden unter Argon und Eiskühlung 1.2 ml (9.50 mMol) Trimethylchlorsilan zugegeben. Das Reaktionsgemisch wurde 30 Minuten am Rückfluss erhitzt, abgekühlt, bei 0° mit 1.7 ml (9.92 mMol) Aethyldiisopropylamin versetzt, anschliessend 15 Minuten bei Raumtemperatur und 2 Stunden unter Rückfluss gerührt, dann auf 0° gekühlt und schliesslich mit einer Lösung von 650 mg (2.98 mMol) Di-tert.butyl-dicarbonat in 0.6 ml Dichlormethan versetzt. Das Reaktionsgemisch wurde 40 Stunden bei Raumtemperatur und 7 Stunden unter Rückfluss gerührt, dann abgekühlt und anschliessend auf eine Mischung von 40 ml gesättigter Natriumhydrogencarbonat-Lösung und Diäthyläther gegossen. Die organische Phase wurde abgetrennt und zweimal mit 30 ml gesättigter Natriumhydrogencarbonat-Lösung extrahiert. Die vereinigten Wasserphasen wurden unter Eiskühlung auf pH 4 gestellt und dreimal mit 100 ml Essigester extrahiert. Die vereinigten organischen Phasen wurden über $Na_2SO_4$ getrocknet und eingeengt, worauf nach Trocknen am Hochvakuum 820 mg (99.6%) (S)-2-(tert.Butoxycarbonylamino)-8-hydroxy-1,2,3,4-tetrahydro-naphthalin-2-carbonsäure als amorpher Feststoff erhalten wurden. $[\alpha]_D$ = +66.0° (c=1.0, Chloroform). IR(KBr): 3403m(br.), 3033w, 2979w, 2933w, 1717s, 1695s, 1589w, 1500w, 1467m, 1395m, 1368m, 1278m, 1164m, 1063w, 776w.

Beispiel 2.9.a

Eine Lösung von 465 mg (1.45 mMol) (R)-2-(tert.Butoxycarbonylamino)-8-hydroxy-1,2,3,4-tetrahydro-naphthalin-2-carbonsäure-methylester in 4.5 ml Tetrahydrofuran/Methanol/Wasser (3:1:1) wurde unter Eiskühlung mit 647 mg Lithiumhydroxid • $1H_2O$ versetzt. Das Reaktionsgemisch wurde 30 Minuten bei 0° und 2 Stunden bei Raumtemperatur gerührt und dann auf eine Mischung von Chloroform/Methanol (6:1) und 0.5N Salzsäure/Eis gegossen. Die organische Phase wurde abgetrennt, über $MgSO_4$ getrocknet und eingeengt, worauf nach Trocknen am Hochvakuum 410 mg (92%) (R)-2-(tert.Butoxycarbonylamino)-8-hydroxy-1,2,3,4-tetrahydro-naphthalin-2-carbonsäure als amorpher Feststoff erhalten wurden. $[\alpha]_D$ = -65.0° (c=0.5, Chloroform).

Beispiel 2.10.b

Eine Suspension von 770 mg (250 mMol) (S)-2-(tert.Butoxycarbonylamino)-8-hydroxy-1,2,3,4-tetrahydro-naphthalin-2-carbonsäure in 5 ml Toluol wurde mit 1.80 ml (7.52 mMol) N,N-Dimethylformamid-di-tert.butylacetal versetzt. Das Reaktionsgemisch wurde während 6 Stunden bei 70° gerührt und dann abgekühlt, worauf nochmals 0.6 ml (2.50 mMol) N,N-Dimethylformamid-di-tert.butylacetal zugegeben und 3 Stunden bei 70° gerührt wurde. Das Reaktionsgemisch wurde auf eine Mischung von Eiswasser und Essigester gegossen, worauf gesättigte Kochsalzlösung zugegeben wurde. Die Wasserphase wurde abgetrennt und mit Essigester extrahiert. Die vereinigten organischen Phasen wurden über $Na_2SO_4$ getrocknet und eingeengt. Der Rückstand wurde an 170 g Kieselgel mit Essigester/Hexan (1:4) chromatographiert, worauf nach Trocknen 665 mg (73%) (S)-2-(tert.Butoxycarbonylamino)-8-hydroxy-1,2,3,4-tetrahydro-naphthalin-2-carbonsäure-tert.butylester als amorpher Feststoff erhalten wurden. $[\alpha]_D$ = +65.5° (c=0.2, Chloroform). IR(KBr): 3406m(br.), 2978m, 2932w, 1721s, 1695s, 1590m, 1497m, 1467m, 1394m, 1368s, 1303m, 1254m, 1162s, 1086m, 784w.

Beispiel 2.11.b

Zu einer Lösung von 800 mg (2.60 mMol) (S)-2-(tert.Butoxycarbonylamino)-8-hydroxy-1,2,3,4-tetrahydro-naphthalin-2-carbonsäure in 6 ml N,N-Dimethylformamid wurden unter Eiskühlung 426 µl (2.86 mMol) 1,8-Diazabicyclo[5.4.2.0]undec-7-en und 340 µl (2.86 mMol) Benzylbromid zugegeben. Das Reaktionsgemisch wurde während 18 Stunden bei Raumtemperatur gerührt und dann auf Eiswasser gegossen, worauf mit Essigsäureäthylester erschöpfend extrahiert wurde. Die vereinigten organischen Phasen wurden mit gesättigter Kochsalzlösung gewaschen, über Magnesiumsulfat getrocknet und eingeengt. Der Rückstand wurde an 120 g Kieselgel mit Hexan/Essigsäureäthylester (7:3) chromatographiert, worauf nach Trocknen am Hochvakuum 829 mg (80.5%) (S)-(tert.Butoxycarbonylamino)-8-hydroxy-1,2,3,4-tetrahydro-naphthalin-2-carbonsäure-benzylester als weisser Schaum erhalten wurden. Smp. 51-65° (Sintern). $[\alpha]_D$ = +64.5° (c=0.2, Chloroform).

Beispiel 2.12.a

Eine Lösung von 440 mg (1.43 mMol) (R)-2-(tert.Butoxycarbonylamino)-8-hydroxy-1,2,3,4-tetrahydro-naphthalin-2-carbonsäure in 8 ml Dichlormethan wurde mit 140 µl Phenyldiazomethan versetzt. Das Reaktionsgemisch wurde 1 Stunde bei Raumtemperatur gerührt und dann eingeengt. Der Rückstand wurde an 50 g Kieselgel mit Chloroform chromatographiert, worauf 384 mg (70%) (R)-2-(tert.Butoxycarbonylamino)-8-hydroxyl-1,2,3,4-tetrahydro-naphthalin-2-carbonsäure-benzylester als amorpher Feststoff erhalten wurden. Smp. 56-60°. $[\alpha]_D$ = -62.7° (c=1, Chloroform).

Beispiel 3.1.b

Zu einer Lösung von 200 mg (0.62 mMol) (S)-2-(tert.Butoxycarbonylamino)-8-hydroxy-1,2,3,4-tetrahydro-naphthalin-2-carbonsäure-methylester in 4 ml Trifluoräthanol wurden unter Argon und Eiskühlung portionenweise 110 mg (0.62 mMol) N-Bromsuccinimid zugegeben. Das Reaktionsgemisch wurde je 1 Stunde bei 0° und bei Raumtemperatur gerührt und dann auf eine Mischung von gesättigter Natriumhydrogencarbonat-Lösung und Chloroform gegossen. Die organische Phase wurde abgetrennt, mit Kochsalzlösung gewaschen, über $MgSO_4$ getrocknet und eingeengt. Der Rückstand wurde an 60 g Kieselgel mit Essigester/Hexan (1:9→4:6) chromatographiert, worauf nach Trocknen am Hochvakuum 220 mg (88.6%) (S)-5-Brom-2-(tert.butoxycarbonylamino)-8-hydroxy-1,2,3,4-tetrahydro-naphthalin-2-carbonsäure-methylester erhalten wurden. Smp. >180° (Zers.). MS: 400 ($M^{+\bullet}$, <1), 343(2), 284, 282(100), 144(52), 57(96).

Beispiel 4.1.1.a

Eine Lösung von 496 mg (1.30 mMol) (R)-2-tert.Butoxycarbonylamino-8-hydroxy-1,2,3,4-tetrahydronaphthalin-2-carbonsäure-benzylester in 1 ml Pyridin wurde unter Eiskühlung und Argon mit 59.5 mg (1.37 mMol) Natriumhydrid-Dispersion (55%) versetzt. Das Gemisch wurde 30 Minuten bei 0° gerührt, mit 748 mg (3.64 mMol) Kupferbromid-dimethylsulfid-Komplex und mit 971 mg (3.90 mMol) 2-Jodnitrobenzol versetzt, auf Raumtemperatur gebracht, 3 Stunden bei 80° und 4 Stunden bei 120° gerührt, abgekühlt und auf eine Mischung von Eis, 0.5N Salzsäure und Essigester gegossen. Die organische Phase wurde abgetrennt, mit 0.5N Salzsäure und gesättigter Natriumchloridlösung gewaschen, über $MgSO_4$ getrocknet und eingeengt. Der Rückstand wurde an Kieselgel (80 g) mit Essigester/Hexan (5:95 → 1:1) chromatographiert, worauf nach Trocknen am Hochvakuum 284 mg (44.8%) (R)-2-tert.Butoxycarbonylamino-8-(2-nitrophenoxy)-1,2,3,4-tetrahydronaphthalin-2-carbonsäure-benzylester als beiger amorpher Festkörper erhalten wurden. $[\alpha]_D$ = -52.3° (c=0.7, Chloroform). MS: 427 (M-Bu; 5), 91(100), 57(46), 292(41), 283(25), 401(16).

Beispiel 4.1.2.a

Zu einer vorhydrierten Suspension von 30 mg Raney-Nickel in 1 ml Methanol wurde eine Lösung von 257 mg (0.49 mMol) (R)-2-tert.Butoxycarbonylamino-8-(2-nitrophenoxy)-1,2,3,4-tetrahydronaphthalin-2-carbonsäure-benzylester in 10 ml Methanol zugegeben, worauf 45 Minuten bei Raumemperatur hydriert wurde. Das Gemisch wurde über Celite filtriert und eingeengt, worauf nach Chromatographie an 50 g Kieselgel mit Essigester/Hexan (1:9 → 1:4) und Trocknung am Hochvakuum 182 mg (75.2%) (R)-8-(2-Aminophenoxy)-2-tert.butoxycarbonylamino-1,2,3,4-tetrahydronaphthalin-2-carbonsäure-benzylester als weisser Festkörper erhalten wurden. Smp. 58-60°. $[\alpha]_D$ = -55.6° (c=0.2, Chloroform).

Beispiel 4.1.3.a

Ein Gemisch von 169 mg (0.35 mMol) (R)-8-(2-Aminophenoxy)-2-tert.-butoxycarbonylamino-1,2,3,4-tetrahydronaphthalin-2-carbonsäurebenzylester und 423.5 mg (1.04 mMol) Fmoc-L-alanin N-hydroxysuccinimidester in 2 ml Tetrahydrofuran wurde 3 Tage bei 50° gerührt und dann eingeengt. Der Rückstand wurde an 30 g Kieselgel mit Hexan/Essigester (95:5 → 4:1) chromatographiert, worauf nach Trocknung am Hochvakuum 100 mg (37%) (R)-2-tert.Butoxycarbonylamino-8-[2-[(S)-2-9H-fluoren-9-ylmethoxycarbonylamino-propionylamino]-phenoxy]-1,2,3,4-naphthalin-2-carbonsäure-benzylester als beiger Festkörper erhalten wurden. Smp. 121-124°. $[\alpha]_D$ = -44.0° (c=0.2, Chloroform).

Beispiel 4.1.4.a

Zu einer Lösung von 95 mg (0.121 mMol) (R)-2-tert.Butoxycarbonylamino-8-[2-[(S)-2-9H-fluoren-9-ylmethoxycarbonylamino-propionylamino]- phenoxy]-1,2,3,4-naphthalin-2-carbonsäure-benzylester in 2 ml N,N-Dimethylformamid wurden 2 Tropfen Diäthylamin zugegeben, worauf 15 Stunden bei Raumtemperatur gerührt wurde. Das Reaktionsgemisch wurde eingeengt, und der Rückstand wurde an 20 g Kieselgel mit Dichlormethan chromatographiert, worauf nach Trocknung 48 mg (71%) (R)-8-[2-[(S)-2-Amino-propionylamino]-phenoxy]-2-tert.butoxycarbonylamino-1,2,3,4-tetrahydro-naphthalin-2-carbonsäure-benzylester als weisser Festkörper erhalten wurden. Smp. 60-62°. $[\alpha]_D$ = -3.5° (c=0.2, Chloroform).

Beispiel 4.1.5.a

Zu einer Lösung von 41 mg (0.073 mMol) (R)-8-[2-[(S)-2-Aminopropionylamino]-phenoxy]-2-tert.butoxycarbonylamino-1,2,3,4-tetrahydronaphthalin-2-carbonsäure-benzylester, 24.5 mg (0.11 mMol) Z-L-Alanin, 29 mg (0.22 mMol) 1-Hydroxybenzotriazol und 21 mg (0.11 mMol) N-Aethyl-N'-(3-dimethylaminopropyl)carbodiimid-hydrochlorid in 1 ml N,N-Dimethylformamid wurden bei 0° 18 µl (0.11 mMol) Aethyldiisopropylamin zugegeben, worauf 3 Stunden bei Raumtemperatur gerührt wurde. Das Reaktionsgemisch wurde auf Wasser und gesättigte Natriumchlorid-Lösung gegossen, worauf das pH auf 3 gestellt und mit Chloroform extrahiert wurde. Die organische Phase wurde eingeengt, und der Rückstand wurde an 10 g Kieselgel mit Chloroform/Methanol (98:2 → Chloroform) chromatographiert, worauf nach Trocknung 36.0 mg (64%) (R)-8-[2-[(S)-2-[(S)-2-Benzyloxycarbonylamino-propionylamino]-propionylamino]-phenoxy]-2-tert.butoxycarbonylamino-1,2,3,4-tetrahydro-naphthalin-2-carbonsäure-benzylester als weisser Festkörper erhalten wurden. $[\alpha]_D$ = -62.0° (c=0.2, Chloroform). MS: (ISP) 403.6 ($MH^+$).

Beispiel 4.1.6.a

Zu einer Lösung von 32.0 mg (0.042 mMol) (R)-8-[2-[(S)-2-[(S)-2-Benzyloxycarbonylamino-propionylamino]-propionylamino]-phenoxy]-2-tert.butoxycarbonylamino-1,2,3,4-tetrahydro-naphthalin-2-carbonsäurebenzylester in 3 ml 2,2,2-Trifluoräthanol wurden 10 mg Palladium-Kohle (10%) zugegeben, worauf 2 Stunden bei Raumtemperatur hydriert wurde. Die Suspension wurde über Watte filtriert und eingeengt. Der Rückstand wurde mehrmals mit 2,2,2-Trifluoräthanol aufgeschlämmt, abfiltriert und am Hochvakuum getrocknet, worauf 24 mg (>100%) (R)-8-[2-[(S)-2-[(S)-2-Amino-propionylamino]-propionylamino]-phenoxy]-2-tert.butoxycarbonylamino-1,2,3,4-tetrahydro-naphthalin-2-carbonsäure als beiger Festkörper erhalten wurden. Smp. 210-212°. MS: (LDP) 541.8 ($MH^+$; 9) 563.7 ($M+Na^+$; 100).

Beispiel 4.1.7.a

Zu einer Lösung von 24.0 mg (0.044 mMol) (R)-8-[2-[(S)-2-[(S)-2-Aminopropionylamino]-propionylamino]-phenoxy]-2-tert.butoxycarbonylamino-1,2,3,4-tetrahydro-naphthalin-2-carbonsäure und 42.5 mg (0.132 mMol) 1,1,3,3-Tetramethyl-2-[1H]-1,2,3-triazolo[5,4-b]pyridin-1-yl]uroniumtetrafluorborat (TATU) in 133 ml N,N-Dimethylformamid wurden bei 0° 18.6 mg (0.22 mMol) getrocknetes $NaHCO_3$ zugegeben, worauf 4 Stunden bei 0° gerührt wurde. Das Lösungsmittel wurde am Hochvakuum abdestilliert, und der Rückstand wurde in Chloroform und Wasser aufgenommen, worauf die organische Phase mit Wasser gewaschen, mit $MgSO_4$ getrocknet und eingeengt wurde. Der Rückstand wurde an 10 g Kieselgel mit Chloroform → Chloroform/Methanol (95:5) chromatographiert, worauf nach Trocknung 14.2 mg (61.7%) (12S,15S,18R)-(12,15-Dimethyl-11,14,17-trioxo-10,11,12,13,14,15,16,17,18,19,20,21-dodecahydro-1,18-etheno-dibenz[b,n][1,5,8,11]oxatriazacyclopentadecin-18-yl)-carbamidsäurer-tert.butylester als weisser Festkörper erhalten wurden. MS: (LDP) 522.6 (4), 545.5 ($M+Na^+$; 100).

Beispiel 4.1.8.a

Zu einer Lösung von 12.5 mg (0.024 mMol) (12S,15S,18R)-(12,15-Dimethyl-11,14,17-trioxo-10,11,12,13,14,15,16,17,18,19,20,21-dodecahydro-1,18-etheno-dibenz[b,n][1,5,8,11]oxatriazacyclopentadecin-18-yl)-carbamidsäurer-tert.butylester in 0.2 ml Dichlormethan wurden bei 0° 0.2 ml Trifluoressigsäure zugetropft, worauf 2 Stunden bei 0° gerührt wurde. Das Reaktionsgemisch wurde zur Trockene eingeengt. Der Rückstand wurde am Hochvakuum getrocknet, aus Dichlormethan umgefällt, abfiltriert und am Hochvakuum getrocknet, worauf 13.1 mg (100%) (12S,15S,18R)-18-Amino-12,15-dimethyl-10,11,12,13,14,15,16,17,18,19,20,21-dodecahydro-1,18-etheno-dibenz[b,n-][1,5,8,11]oxatriazacyclopentadecin-11,14,17-trion-trifluoracetat (1:1) erhalten wurden. MS: (LDP) 445.2 ($M+Na^+$; 100).

Beispiel 4.2.1.a

Zu einer Lösung von 374 mg (0.98 mMol) (R)-2-tert.Butoxycarbonylamino-8-hydroxy-1,2,3,4-tetrahydronaphthalin-2-carbonsäure-benzylester in 3 ml Dioxan wurden bei 0° und unter Argon 52 mg (1.2 mMol) Natriumhydrid-Dispersion (55%) zugegeben, worauf 30 Minuten gerührt wurde. Danach wurden 63 mg (20 Mol%) Tris-[2-(2-methoxyäthoxy)-äthyl]amin (TDA I) und 311 mg (1.96 mMol) 2-Chlor-3-nitropyridin zugegeben, worauf 1 Stunde bei 120° gerührt wurde. Das Reaktionsgemisch wurde auf gesättigte Ammoniumchlorid-Lösung und Essigester gegossen. Die organische Phase wurde mit gesättigter NaCl-Lösung gewaschen, mit $Na_2SO_4$ getrocknet und eingeengt. Der Rückstand wurde an 50 g Kieselgel mit Chloroform chromatographiert, worauf nach Trocknung 328 mg (65%) (R)-2-tert.Butoxycarbonylamino-8-(3-nitro-2-pyridoxy)-1,2,3,4-tetrahydronaphthalin-2-carbonsäure-benzylester als beiger Festkörper erhalten wurden. Smp. 64-66°. MS: 463 (M- =<, 1), 402 (24), 293 (21), 284 (36), 250 (21), 145 (17), 91 (100), 57 (85).

Beispiel 4.2.2.a

Zu einer vorhydrierten Suspension von 100 mg Raney-Nickel in 2 ml Methanol wurde eine Lösung von 300 mg (0.577 mMol) (R)-2-tert.Butoxycarbonylamino-8-(3-nitro-2-pyridoxy)-1,2,3,4-tetrahydronaphthalin-2-carbonsäure-benzylester in 10 ml Methanol gegeben, worauf 1 Stunde hydriert wurde. Das Gemisch wurde über Celite filtriert und eingeengt, und der Rückstand wurde in 5 ml Dichlormethan gelöst, worauf bis zur konstanten Rotfärbung Phenyldiazomethan zugegeben wurde. Das Lösungsmittel wurde abgedampft, und der Rückstand wurde an 50 g Kieselgel mit Essigester/Hexan (1:3) chromatographiert, worauf nach Trocknung am Hochvakuum 256 mg (90.5%) (R)-8-(3-Amino-2-pyridoxy)-2-tert.butoxycarbonylamino-1,2,3,4-tetrahydronaphthalin-2-carbonsäure-benzylester als weisser amorpher Feststoff erhalten wurden. Smp. 71-73°. $[\alpha]_D$ = -37.9° (c=1, Chloroform).

Beispiel 4.2.3.a

Zu einer Lösung von 239 mg (0.49 mMol) (R)-8-(3-Amino-2-pyridoxy)-2-tert.butoxycarbonylamino-1,2,3,4-tetrahydronaphthalin-2-carbonsäurebenzylester in 1 ml Tetrahydrofuran wurden 600 mg (1.47 mMol) Fmoc-L-Alanin N-hydroxysuccinimidester zugegeben, worauf 32 Stunden bei 50° gerührt wurde. Das Gemisch wurde eingeengt, der Rückstand wurde in Wasser und Essigester aufgenommen, die organische Phase wurde abgetrennt, mit $H_2O$ gewaschen, mit $Na_2SO_4$ getrocknet und eingeengt, und der Rückstand wurde an 50 g Kieselgel mit Hexan/Isopropanol (9:1) chromatographiert, worauf nach Trocknung am Hochvakuum 268 mg (70%) (R)-2-tert.Butoxycarbonylamino-8-[3-[(S)-2-9H-fluoren-9-ylmethoxycarbonyl amino]-pyridin-2-yloxy]-1,2,3,4-tetrahydronaphthalin-2-carbonsäure-benzylester als beiger Festkörper erhalten wurden. Smp. 125-130°. $[\alpha]_D$ = -34.8° (c=1, Chloroform).

Beispiel 4.2.4.a

In Analogie zu Beispiel 4.1.4.a wurden 247 mg (0.315 mMol) (R)-2-tert.Butoxycarbonylamino-8-[3-[(S)-2-9H-fluoren-9-ylmethoxycarbonylamino]-pyridin-2-yloxy]-1,2,3,4-tetrahydronaphthalin-2-carbonsäure-benzylester in 4 ml N,N-Dimethylformamid mit 0.6 ml Diäthylamin umgesetzt und der Rückstand an 40 g Kieselgel mit Dichlormethan chromatographiert, worauf nach Trocknung am Hochvakuum 179 mg (R)-8-[3-[(S)-2-Aminopropionylamino]pyridin-2-yloxy]-2-tert.butoxycarbonylamino-1,2,3,4-tetrahydronaphthalin-2-carbonsäure-benzylester erhalten wurden.

Zu 169 mg (0.30 mMol) dieses Produkts, gelöst in 2 ml Tetrahydrofuran, wurden 144 mg (0.45 mMol) Z-L-Alanin-N-hydroxysuccinimidester gegeben, worauf 3 Stunden bei Raumtemperatur gerührt wurde. Das Reaktionsgemisch wurde eingeengt, und der Rückstand wurde an 50 g Kieselgel mit Hexan/Essigester (1:4 → 1:1) chromatographiert, worauf nach Trocknung am Hochvakuum 181 mg (78.4%) (R)-8-[3-[(S)-2-[(S)-2-Benzyloxycarbonylamino-propionylamino]-propionylamino]-pyridin-2-yloxy]-2-tert.butoxycarbonylamino-1,2,3,4-tetrahydro-naphthalin-2-carbonsäure-benzylester als weisses Pulver erhalten wurden. Smp. 172-176°. $[\alpha]_D$ = -59.8° (c=1, Chloroform).

Beispiel 4.2.5.a

Zu einer Lösung von 168 mg (0.22 mMol) (R)-8-[3-[(S)-2-[(S)-2-Benzyloxycarbonylamino-propionylamino]-propionylamino]-pyridin-2-yloxy]-2-tert.butoxycarbonylamino-1,2,3,4-tetrahydro-naphthalin-2-carbonsäure-benzylester in 10 ml Methanol wurden 30 mg Palladium-Kohle (10%) zugegeben, worauf 2 Stunden bei Raumtemperatur hydriert wurde. Das Reaktionsgemisch wurde über Celite filtriert, das Filtrat wurde eingeengt, und der feste Rückstand wurde unter gutem Rühren in Diäthyläther suspendiert, abfiltriert und am Hochvakuum getrocknet, worauf 120 mg (~100%) (R)-8-[3-[(S)-2-[(S)-2-Amino-propionylamino]-propionylamino]-pyridin-2-yloxy]-2-tert.butoxycarbonylamino-1,2,3,4-tetrahydro-naphthalin-2- carbonsäure als weisses Pulver erhalten wurden. Smp. 239-241°. MS: (ISN) 540.2, 100% ($MH^-$), 596.0, (53), ($MH^-$) (Ni).

Beispiel 4.2.6.a

Zu einer Lösung von 81 mg (0.149 mMol) (R)-8-[3-[(S)-2-[(S)-2-Aminopropionylamino]-propionylamino]-pyridin-2-yloxy]-2-tert.butoxycarbonylamino-1,2,3,4-tetrahydro-naphthalin-2-carbonsäure in 250 ml N,N-Dimethylformamid wurden bei 0° 170 mg (0.447 mMol) O-Benzotriazol-1-yl-N,N,N',N'-tetramethyl-uroniumhexafluorphosphat (HBTU) und 62 mg (0.745 mMol) getrocknetes $NaHCO_3$ zugegeben, worauf 3 Stunden bei 0° gerührt wurde. Das Reaktionsgemisch wurde eingeengt, der Rückstand wurde in Wasser und Essigester aufgenommen, die organische Phase wurde dreimal mit gesättigter $NaHCO_3$-Lösung gewaschen, über $Na_2SO_4$ getrocknet und eingeengt, und der Rückstand wurde an 30 g Kieselgel mit Chloroform/Methanol (98:2) chromatographiert, worauf nach Trocknung am Hochvakuum 50 mg (64%) (12S,15S,18R)-(12,15-Dimethyl-11,14,17-trioxo-10,11,12,13,14,15,16,17,18,19,20,21-dodecahydro-1,18-etheno-pyrido[2,3-b]benz[n][1,4,7,10]oxatriazacyclopentadecin-18-yl)-carbamidsäure-tert.-butylester als weisses Pulver erhalten wurden. Smp. (Zers.) = 265°. $[\alpha]_D$ = -127.2° (c=0.5, Chloroform).

Beispiel 4.2.7.a

Zu einer Lösung von 50.6 mg (0.095 mMol) (12S,15S,18R)-(12,15-Dimethyl-11,14,17-trioxo-10,11,12,13,14,15,16,17,18,19,20,21-dodecahydro-1,18-etheno-pyrido[2,3-b]benz[n][1,4,7,10]oxatriazacyclopentadecin-18-yl)-carbamidsäure-tert.butylester in 3 ml Dichlormethan wurden bei 0° 3 ml Trifluoressigsäure zugetropft, worauf 3 Stunden bei 0° gerührt wurde. Das Gemisch wurde am Hochvakuum eingeengt, und der Rückstand wurde in Diäthyläther/Hexan (1:1) suspendiert, abfiltriert und am Hochvauum getrocknet, worauf 42.0 mg (81.8%) (12S,15S,18R)-18-Amino-12,15-dimethyl-10,11,12,13,14,15,16,17,18,19,20,21-dodecahydro-1,18-etheno-pyrido[2,3-b]benz[n][1,4,7,10]-oxatriazacyclopentadecin-11,14,17-trion-trifluoracetat (1:1) als weisses Pulver erhalten wurden. Smp. (Zers.) = 212-217°. $[\alpha]_D$ = -50.2° (c=1.0, Chloroform).

Beispiel 4.3.1.a

Zu einer Lösung von 514 mg (1.6 mMol) (R)-2-tert.Butoxycarbonylamino)-8-hydroxy-1,2,3,4-tetrahydronaphthalin-2-carbonsäure-methylester in 2 ml wasserfreiem Dioxan wurden bei 0° unter Argon 74 mg (1.68 mMol) Natriumhydrid-Dispersion (55%) gegeben, worauf 20 Minuten bei 0° gerührt wurde. Zum Reaktionsgemisch wurden anschliessend 100 mg (20 Mol%) Tris-[2-(2-methoxyäthoxy)-äthyl]amin (TDA I) und 1.28 g (4.8 mMol) (S)-1-Brom-3-tert.butyldimethylsiloxy-2-methylpropan gegeben, worauf 30 Minuten bei 120° im Bombenrohr gerührt wurde. Danach wurde das Reaktionsgemisch abgekühlt, mit 14.8 mg Natriumhydrid-Dispersion versetzt und nochmals 30 Minuten bei 120° gerührt. Nach Abkühlen wurde das Reaktionsgemisch auf Eiswasser und Essigester gegossen; die organische Phase wurde mit Wasser und gesättigter NaCl-Lösung gewaschen, über $Na_2SO_4$ getrocknet und eingeengt, und der Rückstand wurde an 120 g Kieselgel mit Hexan/Essigester (9:1 → 4:1) chromatographiert, worauf nach Trocknung am Hochvakuum 312 mg (38.4%) (R)-2-tert.Butoxycarbonylamino-8-[(S)-(3-tert.butyldimethylsiloxy-2-methyl-1-propyloxy)]-1,2,3,4-tetrahydronaphthalin-2-carbonsäuremethylester als farbloses Oel erhalten wurden. $[\alpha]_D$ = -70.6° (c=1.0, Chloroform). MS (ISP): 525.6 ($M+NH_4^+$; 100), 508.6 ($M+H^+$; 50).

Beispiel 4.3.2.a

Zu einer Lösung von 300 mg (0.59 mMol) (R)-2-tert.Butyloxycarbonylamino-8-[(S)-(3-tert.butyldimethylsilyoxy-2-methyl-1-propyloxy)]-1,2,3,4-tetrahydronaphthalin-2-carbonsäure-methylester in 2 ml Tetrahydrofuran wurden bei 0° 560 mg (1.77 mMol) Tetrabutylammoniumfluorid zugegeben, worauf 3 Stunden gerührt wurde. Das Reaktionsgemisch wurde auf Eiswasser und Essigester gegossen, die organische Phase wurde mit Wasser gewaschen, mit $Na_2SO_4$ getrocknet und eingeengt, und der Rückstand wurde an 40 g Kieselgel mit Essigester/Hexan (1:4 → 1:1) chromatographiert, worauf nach Trocknung am Hochvakuum 183 mg (78.7%) (R)-2-tert.Butoxycarbonylamino-8-[(S)-(3-hydroxy-2-methylpropyloxy)]-1,2,3,4-tetrahydronaphthalin-2-carbonsäure-methylester als farbloses Oel erhalten wurden. MS (ISP): 411.5 ($M+NH_4^+$; 100), 394.4 ($M+H^+$; 62), 338.4 (13).

Beispiel 4.3.3.a

Zu einer Lösung von 179 mg (0.455 mMol) (R)-2-tert.Butoxycarbonylamino-8-[(S)-3-hydroxy-2-methylpropyloxy)]-1,2,3,4-tetrahydronaphthalin-2-carbonsäure-methylester und 92 mg (0.91 mMol) Triäthylamin in 3 ml Dichlormethan wurden bei 0° unter Argon 95.3 mg (0.5 mMol) Tosylchlorid und eine Spatelspitze 4-(N,N-Dimethylamino)-pyridin zugegeben. Das Reaktionsgemisch wurde 2 Stunden bei 0° und 15 Minuten bei Raumtemperatur gerührt, worauf nochmals 46 mg (0.45 mMol) Triäthylamin und 48 mg (0.25 mMol) Tosylchlorid zugegeben wurden und 2 Stunden bei Raumtemperatur gerührt wurde. Das Reaktionsgemisch wurde auf Eiswasser und Essigester gegossen, die organische Phase wurde mit Wasser gewaschen, über $Na_2SO_4$ getrocknet und eingeengt, und der Rückstand wurde an $SiO_2$ mit Essigester/Hexan (1:4 → 1:1) chromatographiert, worauf nach Trocknung am Hochvakuum 127 mg (51%) (R)-2-tert.Butoxycarbonylamino-8-[(R)-2-methyl-2-(4-methyl-phenylsulfonyloxy)-propoxy]-1,2,3,4-tetrahydronaphthalin-2-carbonsäuremethylester als weisser Festkörper erhalten wurden. Smp. 55-58°. MS (ISP): 565.1 ($M+NH_4^+$ ; 90), 548.1 ($M+H^+$; 100), 448.0 (95).

Beispiel 4.3.4.a

Zu einer Lösung von 120 mg (0.182 mMol) (R)-2-tert.Butoxycarbonylamino-8-[(R)-2-methyl-2-(4-methyl-phenylsulfonyloxy)-propoxy]-1,2,3,4-tetrahydronaphthalin-2-carbonsäure-methylester und 142 mg (2.13 mMol) Natriumazid in 1.5 ml Methanol wurden 25 mg (0.48 mMol) Ammoniumchlorid gegeben, worauf 3 Stunden bei 70° gerührt wurde. Danach wurden nochmals 142 mg (2.19 mMol) Natriumazid und 25 mg Ammoniumchlorid zugegeben und insgesamt 12 Stunden bei 70° gerührt. Das Reaktionsgemisch wurde eingeengt, der Rückstand wurde in Wasser und Essigester aufgenommen, die organische Phase wurde mit Wasser gewaschen, mit $Na_2SO_4$ getrocknet und eingeengt, und der Rückstand wurde an Kieselgel mit Essigester/Hexan (1:9) chromatographiert, worauf nach Trocknung am Hochvakuum 89 mg (97%) (R)-8-[(S)-3-Azido-2-methyl-propoxy]-2-tert.butoxycarbonylamino-1,2,3,4-tetrahydronaphthalinsäuremethylester als farbloses Harz erhalten wurden. MS (ISP): 419.4 ($M+H^+$; 50), 319.2 (100).

Beispiel 4.3.5.a

Zu einer Lösung von 83 mg (0.198 mMol) (R)-8-[(S)-3-Azido-2-methylpropoxy]-2-tert.butoxycarbonylamino-1,2,3,4-tetrahydronaphthalinsäuremethylester in 5 ml Aethanol wurden 50 mg Palladium-Kohle (10%) zugegeben, worauf 2 Stunden bei Raumtemperatur hydriert wurde. Das Reaktionsgemisch wurde über Celite filtriert, und anschliessend wurde nochmals zweimal in der selben Weise hydriert. Die Lösung wurde eingeengt und am Hochvakuum getrocknet, wobei 71 mg eines Gemisches von (R)-8-[(S)-3-Amino-2-methyl-propoxy]-2-tert.butoxycarbonylamino-1,2,3,4-tetrahydronaphthalin-2-carbonsäure-methylester und (R)-2-tert.Butoxycarbonylamino-8-[(S)-3-ethylamino-2-methyl-propoxy]-1,2,3,4-tetrahydronaphthalin-2-carbonsäure-methylester erhalten wurden. MS (ISP): 421.5 ($M+H^+$; 92), 393.5 ($M+H^+$; 100).

Beispiel 4.3.6.a

In Analogie zu Beispiel 4.1.5.a wurden 70 mg (~0.178 mMol) eines Gemisches von (R)-8-[(S)-3-Amino-2-methyl-propoxy]-2-tert.butoxycarbonylamino-1,2,3,4-tetrahydronaphthalin-2-carbonsäure-methylester und (R)-2-tert.Butoxycarbonylamino-8-[(S)-3-ethylamino-2-methyl-propoxy]-1,2,3,4-tetrahydronaphthalin-2-carbonsäure-methylester in 1.5 ml N,N-Dimethylformamid mit 78 mg (0.267 mMol) Z-L-Alanylalanin, 114 mg (0.356 mMol) O-(1,2-Dihydro-2-oxo-1-pyridyl)-N,N,N',N'-tetramethyl-uroniumtetrafluoroborat (TPTU), 54.5 mg (0.356 mMol) 1-Hydroxybenzotriazol und 86 µl (0.534 mMol) Aethyldiisopropylamin 3 Stunden bei 0° umgesetzt. Der Rückstand wurde an 20 g Kieselgel mit Chloroform → Chloroform/Methanol (95:5 → 4:1) chromatographiert, worauf nach Trocknung am Hochvakuum 30 mg (40.3%) (R)-8-[(S)-3-[[(S)-2-[(S)-2-Benzyloxycarbonylamino-propionylamino]-propionyl]-ethyl-amino]-2-methyl-propoxy]-2-tert.-butoxycarbonylamino-1,2,3,4-tetrahydronaphthalin-2-carbonsäure-methylester, [MS (ISP): 697.6 ($M+H^+$; 100)] und dann 26 mg (21.8%) (R)-8-[(S)-3-[(S)-2-[(S)-2-Benzyloxycarbonylamino-propionylamino]-propionylamino]-2-methyl-propoxy]-2-tert.butoxycarbonylamino-1,2,3,4-tetrahydronaphthalin-2-carbonsäure-methylester [MS (ISP): 535.5 ($M+H^+$; 100)] erhalten wurden.

Beispiel 4.3.7.a

Zu einer Lösung von 28.0 g (42. µMol) (R)-8-[(S)-3-[(S)-2-[(S)-2-Benzyloxycarbonylamino-propionylamino]-propionylamino]-2-methyl-propoxy]-2-tert.butoxycarbonylamino-1,2,3,4-tetrahydronaphthalin-2-carbonsäure-methylester in 5 ml 2,2,2-Trifluoräthanol und 2 Tropfen Wasser wurden 15 mg Palladium-Kohle (10%) zugegeben, worauf 1 Stunde bei Raumtemperatur hydriert wurde. Das Reaktionsgemisch wurde über Celite filtriert, das Filtrat wurde eingeengt und der Rückstand wurde am Hochvakuum getrocknet, worauf 19 mg (84.8%) (R)-8-[(S)-3-[(S)-2-[(S)-Aminopropionylamino]-propionylamino]-2-methyl-propoxy]-2-tert.butoxycarbonylamino-1,2,3,4-tetrahydronaphthalin-2-carbonsäure-methylester als farbloser Lack erhalten wurden. MS (ISP): 535.5 ($M+H^+$; 100).

Beispiel 4.3.8.a

Zu einer Lösung von 19.0 mg (35.0 mMol) (R)-8-[(S)-3-[(S)-2-[(S)-Aminopropionylamino]-propionylamino]-2-methyl-propoxy]-2-tert.butoxycarbonylamino-1,2,3,4-tetrahydronaphthalin-2-carbonsäure-methylester in 1 ml Tetrahydrofuran/Methanol/Wasser (3:1:1) wurden 123 µl einer 1N wässrigen Lithiumhydroxid-Lösung zugegeben, worauf 15 Stunden bei Raumtemperatur gerührt wurde. Danach wurden nochmals 200 µl 1N Lithiumhydroxid-Lösung zugegeben und 48 Stunden bei Raumtemperatur gerührt. Das Reaktionsgemisch wurde mittels 2N HCl neutral gestellt und zur Trockene eingeengt. Der Rückstand wurde über eine MCI Gel-Säule mit Wasser → Wasser/Methanol (95:5 → 4:1) chromatographiert, worauf nach Trocknung am Hochvakuum 17 mg (91.9%) (R)-8-[(S)-3-[(S)-2-[(S)-2-Aminopropionylamino]-propionylamino]-2-methyl-propoxy]-2-tert.butoxycarbonylamino-1,2,3,4-tetrahydronaphthalin-2-carbonsäure als weisses Pulver erhalten wurden. MS (ISP): 521.4 ($M+H^+$; 100).

Beispiel 4.3.9.a

Zu einer Lösung von 12.0 mg (23.0 mMol) (R)-8-[(S)-3-[(S)-2-[(S)-2-Amino-propionylamino]-propionylamino]-2-methyl-propoxy]-2-tert.-butoxycarbonylamino-1,2,3,4-tetrahydronaphthalin-2-carbonsäure und 22.0 mg (70.0 mMol) 1,1,3,3-Tetramethyl-2-[1H-1,2,3-triazolo[5,4-b]-pyridin-1-yl]uroniumtetrafluoroborat (TATU) in 70 ml wasserfreiem N,N-Dimethylformamid wurden 9.6 mg (0.115 mMol) $NaHCO_3$ zugegeben, worauf 15 Stunden bei Raumtemperatur gerührt und dann eingeengt wurde. Der Rückstand wurde in Chloroform aufgenommen, die Lösung wurde mit Wasser gewaschen, die organische Phase wurde mit $Na_2SO_4$ getrocknet und eingeengt, und der Rückstand wurde an 10 g Kieselgel mit Chloroform → Chloroform/Methanol (98:2) chromatographiert, worauf nach Trocknung am Hochvakuum 5.9 mg (50.9%) (3S,7S,10S,13R)-(3,7,10-Trimethyl-6,9,12-trioxo-3,4,5,6,7,8,9,10,11,12,13,14,19,20-tetradecahydro-13,15-etheno-2H-1,5,8,11-benzoxatriazacyclohexadecin-13-yl)-carbamidsäure-tert.butylester als weisses Pulver erhalten wurden. MS: 503.2 ($M+H^+$; 55), 91.1 (100), 216.9 (89), 109.1 (33), 149.0 (25).

Beispiel 4.3.10.a

In Analogie zu Beispiel 4.1.8.a wurden 5.9 mg (12.0 mMol) (3S,7S,10S,13R)-(3,7,10-Trimethyl-6,9,12-trioxo-3,4,5,6,7,8,9,10,11,12,13,14, 19,20-tetradecahydro-13,15-etheno-2H-1,5,8,11-benzoxatriazacyclohexadecin-13-yl)-carbamidsäure-tert.butylester in 0.5 ml Dichlormethan und 0.5 ml Trifluoressigsäure bei 0° umgesetzt, worauf der

Rückstand nach Fällen aus Ether/Hexan (1:1) am Hochvakuum getrocknet wurde. Es wurden 6.0 mg (99.0%) (3S,7S,10S,13R)-13-Amino-3,7,10-trimethyl-3,4,5,6,7,8,9,10,11,12,13, 14,19,20-tetradecahydro-13,15-etheno-2H-1,-5,8,11-benzoxatriazacyclohexadecin-6,9,12-trion-trifluoracetat (1:1) als weisses Pulver erhalten. MS (ISP): 403.5 ($M+H^+$; 100).

Beispiel 4.4.1.a

In Analogie zu Beispiel 4.3.7.a wurden 25.0 mg (35.0 mMol) (R)-8-[(S)-3-[[(S)-2-[(S)-2-Benzyloxycarbonylamino-propionylamino]-propionyl]-ethylamino]-2-methyl-propoxy]-2-tert.butoxycarbonylamino-1,2,3,4-tetrahydronaphthalin-2-carbonsäure-methylester in 3 ml 2,2,2-Trifluoräthanol und 15 mg Palladium-Kohle (10%) 2 Stunden bei Raumtemperatur hydriert. Nach Aufarbeitung gemäss Beispiel 4.3.7.a und Trocknung am Hochvakuum wurden 18.0 mg (90%) (R)-8-[(S)-3-[[(S)-2-[(S)-2-Amino-propionylamino]-propionyl]-ethyl-amino]-2-methyl-propoxy]-2-tert.butoxycarbonylamino-1,-2,3,4-tetrahydronaphthalin-2-carbonsäure-methylester als farbloses Oel erhalten. MS (ISP): 563.4 ($M+H^+$; 100).

Beispiel 4.4.2.a

In Analogie zu Beispiel 4.3.8.a wurden 18.0 mg (32.0 mMol) (R)-8-[(S)-3-[[(S)-2-[(S)-2-Amino-propionylamino]-propionyl]-ethyl-amino]-2-methylpropoxy]-2-tert.butoxycarbonylamino-1,2,3,4-tetrahydronaphthalin-2-carbonsäure-methylester in 1 ml Tetrahydrofuran/Methanol/Wasser (3:1:1) mit 320 µl 1N Lithiumhydroxid-Lösung umgesetzt. Nach Aufarbeitung und Reinigung gemäss Beispiel 4.3.8.a wurden 17.0 mg (96.8%) (R)-8-[(S)-3-[(S)-2-[(S)-2-Amino-propionylamino]-propionyl]-ethyl-amino]-2-methyl-propoxy]-2-tert.butoxycarbonylamino-1,2,3,4-tetrahydronaphthalin-2-carbonsäure als weisses Pulver erhalten. MS: 549 ($M+H^+$), 216.9 (100), 91.1 (75), 149.0 (36), 513 (35), 471 (31), 109 (29).

Beispiel 4.4.3.a

In Analogie zu Beispiel 4.1.7.a wurden 18.0 mg (32.0 µMol) (R)-8-[(S)-3-[[(S)-2-[(S)-2-Amino-propionylamino]-propionyl]-ethyl-amino]-2-methyl-propoxy]-2-tert.butoxycarbonylamino-1,2,3,4-tetrahydronaphthalin-2-carbonsäure mit 31.0 mg (99.0 µMol) 1,1,3,3-Tetramethyl-2-[1H-1,2,3-triazolo[5,4-b]-pyridin-1-yl]-uroniumtetrafluoroborat (TATU) und 13.8 mg (0.165 mMol) $NaHCO_3$ in 100 ml wasserfreien N,N-Dimethylformamid während 15 Stunden bei 0° umgesetzt. Nach Aufarbeitung gemäss Beispiel 4.1.7.a, Chromatographie an 5 g Kieselgel mit Chloroform → Chloroform/Methanol (98:2) und nach Trocknung am Hochvakuum wurden 12.9 mg (74.1%) (3S,7S,10S,13R)-(5-Ethyl-3,7,10-trimethyl-6,9,12-trioxo-13,15-etheno-3,4,5,6,7,8,9,10,11,12,13,14,19,20-tetradecahydro-2H-1,5,8,11-benzoxatriazacyclohexadecin-13-yl)-carbamidsäure-tert.butylester als weisses Pulver erhalten. MS (ISP): 531.6 ($M+H^+$; 100), 553.6 ($M+Na^+$; 52).

Beispiel 4.4.4.a

In Analogie zu Beispiel 4.1.8.a wurden 12.9 mg (24.0 µMol) (3S,7S,10S,13R)-(5-Ethyl-3,7,10-trimethyl-6,9,12-trioxo-13,15-etheno-3,4,5,6,7,8,9,10,11,12,13,14,19,20-tetradecahydro-2H-1,5,8,11-benzoxatriazacyclohexadecin-13-yl)-carbamidsäure-tert.butylester in 0.2 ml Dichlormethan und 0.2 ml Trifluoressigsäure bei 0° umgesetzt, worauf gemäss Beispiel 4.1.8.a aufgearbeitet und gereinigt wurde. Der Rückstand wurde in Aether/Hexan (1:1) suspendiert, abfiltriert und am Hochvakuum getrocknet, worauf 10.0 mg (75.8%) (3S,7S,10S,13R)-13-Amino-5-ethyl-3,7,10-trimethyl-3,4,5,6,7,8,9,10,11,12,13,14,19,20-tetradecahydro-2H-1,5,8,11-benzoxatriazacyclohexadecin-6,9,12-trion-trifluoracetat (1:1) als weisses Pulver erhalten wurden. MS (LDP): 453.5 ($M+Na^+$; 100), 469.4 ($M+K^+$, 26), 431.5 ($M+H^+$; 4).

Beispiel 4.5.1.a

Zu einer Lösung von 514 mg (1.60 mMol) (R)-2-tert.Butoxycarbonylamino-8-hydroxy-1,2,3,4-tetrahydronaphthalin-2-carbonsäure-methylester in 2 ml Pyridin wurden unter Argon bei 0° 74 mg (1.68 mMol) Natriumhydrid-Dispersion (55%) zugegeben, worauf 20 Minuten gerührt wurde. Danach wurden 460 mg (2.24 mMol) Kupferbromid-dimethylsulfid-Komplex und 1.54 g (4.8 mMol) Benzyl N-2-bromobenzyl-carbamat bei 0° zugegeben, worauf 4 Stunden auf 120° erwärmt wurde. Das Reaktionsgemisch wurde abgekühlt und auf Eis, 0.5N HCl und Essigester gegossen, die organische Phase wurde mit Wasser gewaschen, mit $Na_2SO_4$ getrocknet und eingeengt, und der Rückstand wurde an 100 g Kieselgel mit Hexan/Essigester (4:1 → 1:1) chromatographiert, worauf nach Trocknung am Hochvakuum 368 mg (41%) (R)-8-(2-Benzyloxycarbonyl-aminomethyl-phenoxy)-2-tert.butyloxycarbonylamino-1,2,3,4-tetrahydronaphthalin-2-carbonsäure-methylester als leicht gelbes Pulver erhalten wurden. Smp. 82-85°. $[\alpha]_D$ = -92.0° (c=1, Chloroform).

Beispiel 4.5.2.a

Eine Lösung von 324 mg (0.58 mMol) (R)-8-(2-Benzyloxycarbonylaminomethyl-phenoxy)-2-tert.butoxycarbonylamino-1,2,3,4-tetrahydronaphthalin-2-carbonsäure-methylester in 4 ml Aethanol wurde mit 100 mg Palladium-Kohle (10%) versetzt, worauf 3 Stunden bei Raumtemperatur hydriert wurde. Das Reaktionsgemisch wurde über Celite filtriert und eingeengt, und der Rückstand wurde an 30 g Kieselgel mit Chloroform → Chloroform/Methanol (95:5) chromatographiert, worauf nach Trocknung am Hochvakuum 211 mg (85.4%) (R)-8-(2-Aminomethyl-phenoxy)-2-tert.butoxycarbonylamino-1,2,3,4-tetrahydronaphthalin-2-carbonsäure-methylester als weisser amorpher Feststoff erhalten wurden. Smp. 57-59°. MS (ISP): 427.6 ($M+H^+$; 100).

Beispiel 4.5.3.a

In Analogie zu Beispiel 4.1.5.a wurden 205 mg (0.48 mMol) (R)-8-(2-Aminomethyl-phenoxy)-2-tert.butoxycarbonylamino-1,2,3,4-tetrahydronaphthalin-2-carbonsäure-methylester in 5 ml N,N-Dimethylformamid mit 212 mg (0.72 mMol) Z-L-Alanyl-L-alanin, 97 mg (0.72 mMol) 1-Hydroxybenzotriazol, 160 mg (0.84 mMol) N-Aethyl-N'-(3-dimethylaminopropyl)-carbodiimid-hydrochlorid und 216 µl (1.26 mMol) Aethyldiisopropylamin während 5 Stunden bei 0° umgesetzt. Das Reaktionsgemisch wurde am Hochvakuum eingeengt, der Rückstand wurde in Wasser und Essigester aufgenommen, die organische Phase wurde mit Wasser gewaschen, mit $Na_2SO_4$ getrocknet und eingeengt, und der Rückstand wurde an 70 g Kieselgel mit Chloroform/Methanol (99:1) chromatographiert, worauf 193 mg (57.2%) (R)-8-[2-[(S)-2-[(S)-Benzyloxycarbonylamino-propionylamino]-propionylaminomethyl]-phenoxy]-2-tert.butoxycarbonylamino-1,2,3,4-tetrahydronaphthalin-2-carbonsäure-methylester als weisser amorpher Festkörper erhalten wurden. Smp. 82-84°. $[\alpha]_D$ = -80.0° (c=1, Chloroform).

Beispiel 4.5.4.a

Zu einer Lösung von 250 mg (0.356 mMol) (R)-8-[2-[(S)-2-[(S)-Benzyloxycarbonylamino-propionylamino]-propionylaminomethyl]-phenoxy]-2-tert.butoxycarbonylamino-1,2,3,4-tetrahydronaphthalin-2-carbonsäure-methylester in 5 ml Aethanol wurden 50 mg Palladium-Kohle (10%) gegeben, worauf 1 Stunde bei Raumtemperatur hydriert wurde. Das Gemisch wurde über Celite filtriert und eingeengt, und der Rückstand wurde an 30 g Kieselgel mit Chloroform/Methanol (95:5) chromatographiert, worauf nach Trocknung am Hochvakuum 169 mg (83.6%) (R)-8-[2-[(S)-[(S)-2-Amino-propionylamino]-propionylaminomethyl]-phenoxy]-2-tert.butoxycarbonylamino-1,2,3,4-tetrahydronaphthalin-2-carbonsäure-methylester als weisser Feststoff erhalten wurden. Smp. 125-130°. $[\alpha]_D$ = -97.0° (c=1.0, Chloroform).

Beispiel 4.5.5.a

Zu einer Lösung von 128 mg (0.225 mMol) (R)-8-[2-[(S)-[(S)-2-Aminopropionylamino]-propionylaminomethyl]-phenoxy]-2-tert.butoxycarbonylamino-1,2,3,4-tetrahydronaphthalin-2-carbonsäure-methylester in 1.5 ml Tetrahydrofuran/Methanol/Wasser (3:1:1) wurde unter Eiskühlung 47 mg (1.12 mMol) Lithiumhydroxid-Monohydrat zugegeben, worauf 24 Stunden bei Raumtemperatur gerührt wurde. Das Reaktionsgemisch wurde eingeengt, der Rückstand wurde in Wasser gelöst, und die Lösung wurde mit 2N HCl neutral gestellt und eingeengt. Der Rückstand wurde an MCI-Gel zunächst mit Wasser und dann mit Methanol/Wasser (1:4) chromatographiert, worauf nach Trocknung am Hochvakuum 106 mg (85%) (R)-8-[2-[(S)-2-[(S)-2-Amino-propionylamino]-propionylaminomethyl]-phenoxy]-2-tert.butoxycarbonylamino-1,2,3,4-tetrahydronaphthalin-2-carbonsäure als weisses Pulver erhalten wurden. Smp. 222-226°. $[\alpha]_D$ = -43.5° (c=0.2, Methanol).

Beispiel 4.5.6.a

Zu einer Lösung von 100 mg (0.18 mMol) (R)-8-[2-[(S)-2-[(S)-2-Aminopropionylamino]-propionylaminomethyl]-phenoxy]-2-tert.butoxycarbonylamino-1,2,3,4-tetrahydronaphthalin-2-carbonsäure und 204 mg (0.54 mMol) O-Benzotriazol-1-yl-N,N,N',N'-tetramethyl-uroniumhexafluorphosphat (HBTU) in 220 ml N,N-Dimethylformamid wurden unter Argon bei 0° 75.6 mg (0.90 mMol) getrocknetes $NaHCO_3$ zugegeben, worauf 4 Stunden bei 0° und 1 Stunde bei Raumtemperatur gerührt wurde. Das Reaktionsgemisch wurde am Hochvakuum eingeengt, und der Rückstand wurde an 20 g Kieselgel mit Chloroform/Methanol (98:2) chromatographiert, worauf 95 mg (~100%) (13S,16S,19R)-(13,16-Dimethyl-12,15,18-trioxo-11,12,13,14,15, 16,17,18,19,20,21,22-dodecahydro-1,19-etheno-10H-dibenz[b,o][1,5,8,11]oxatriazacyclohexadecin-19-yl)-carbamidsäure-tert.butylester als weisses Pulver erhalten wurden. Smp. (Zers.) = 184-186°. $[\alpha]_D$ = +34.0° (c=0.2, Methanol).

Beispiel 4.5.7.a

Zu einer Lösung von 83 mg (0.155 mMol) (13S,16S,19R)-(13,16-Dimethyl-12,15,18-trioxo-11,12,13,14,15,16,17,18,19,20,21,22-dodecahydro-1,19-etheno-10H-dibenz[b,o][1,5,8,11]oxatriazacyclohexadecin-19-yl)-carbamidsäure-tert.butylester in 3 ml Dichlormethan wurden bei 0° 3 ml Trifluoressigsäure zugegeben, worauf 3 Stunden bei 0° gerührt wurde. Das Lösungsmittel wurde am Hochvakuum entfernt, und der Rückstand wurde an 15 g Kieselgel mit Chloroform → Chloroform/Methanol (95:5) chromatographiert, worauf nach Trocknung am Hochvakuum 71 mg (83.4%) (13S,16S,19R)-19-Amino-13,16-dimethyl-11,12,13,14,15,16,17,18,19,20,21,22-dodecahydro-1,19-etheno-10H-dibenz[b,o][1,5,8,11]oxatriazacyclohexadecin-12,15,18-trion-trifluoracetat (1:1) als weisses Pulver erhalten wurden. Smp. 185-188°. MS (ISP): 437.4 ($M+H^+$; 100).

Beispiel 5.1.1.a

Zu einer Lösung von 10.0 mg (0.018 mMol) (12S,15S,18R)-18-Amino-12,15-dimethyl-10,11,12,13,14,15,16,17,18,19,20,21-dodecahydro-1,18-etheno-dibenz[b,n][1,5,8,11]oxatriazacyclopentadecin-11,14,17-trion-trifluoracetat (1:1), 6.8 mg (0.036 mMol) Boc-L-Alanin, 6.5 mg (0.048 mMol) 1-Hydroxy-7-pyridinotriazol (HOAT) und 11.5 mg (0.036 mMol) 1,1,3,3-Tetramethyl-2-[1H-1,2,3-triazolo[5,4-b]pyridin-1-yl]-uroniumtetrafluorborat (TATU) in 0.2 ml N,N-Dimethylformamid wurden unter Eiskühlung und Argon 14.0 µl (0.084 mMol) Aethyldiisopropylamin zugegeben, worauf 15 Stunden bei 0° gerührt wurde. Das Reaktionsgemisch wurde eingeengt, der Rückstand wurde in Wasser und Chloroform aufgenommen, die organische Phase wurde mit Wasser gewaschen und eingeengt, und der Rückstand wurde an 10 g Kieselgel mit Chloroform → Chloroform/Methanol (98:2) chromatographiert, worauf nach Trocknung 10.0 mg (90.4%) (S)-1-[(12S,15S,18R)-12,15-Dimethyl-11,14,17-trioxo-10,11,12,13,14,15-,16,17,18,19,20,21-dodecahydro-1,18-etheno-dibenz[b,n][1,5,8,11]oxatriazacyclopentadecin-18-ylcarbamoyl]-ethylcarbamidsäure-tert.butylester als weisser Festkörper erhalten wurden. MS (LDP): 616.4 ($M+Na^+$; 100), 322.3 (52), 478.3 (50), 623.3 (43), 443.2 (41).

Beispiel 5.1.2.a

Zu einer Lösung von 9.5 mg (0.016 mMol) (S)-1-[(12S,15S,18R)-12,15-Dimethyl-11,14,17-trioxo-10,11,12,13,14,15,16,17,18,19,20,21-dodecahydro-1,18-etheno-dibenz[b,n][1,5,8,11]oxatriazacyclopentadecin-18-ylcarbamoyl]-ethylcarbamidsäure-tert.butylester in 0.1 ml Dichlormethan wurde bei 0° unter Argon 0.1 ml Trifluoressigsäure zugegeben, worauf 3 Stunden bei 0° gerührt wurde. Das Lösungsmittel wurde am Hochvakuum abgezogen, und der Rückstand wurde getrocknet und mit Dichlormethan umgefällt. Nach Trocknung erhielt man 11.0 mg (~100%) (S)-2-Amino-N-[(12S,15S,18R)-12,15-dimethyl-11,14,17-trioxo-10,11,12,13,14,15,16,17,18,19,20,21-dodecahydro-1,18-etheno-dibenz[b,n][1,5,8,11]-oxatriazacyclopentadecin-18-yl]-propionamid-trifluoracetat (1:1) als weisses Pulver. MS (LDP): 494.6 ($MH^+$; 8), 516.5 ($M+Na^+$; 100), 532.4 ($M+K^+$; 34).

Beispiel 5.1.3.a

Zu einer Lösung von 11.0 mg (0.016 mMol) (S)-2-Amino-N-[(12S,15S,18R)-12,15-dimethyl-11,14,17-trioxo-10,11,12,13,14,15,16,17,18, 19,20,21-dodecahydro-1,18-etheno-dibenz[b,n][1,5,8,11]-oxatriazacyclopentadecin-18-yl]-propionamid-trifluoracetat (1:1), 4.3 mg (0.032 mMol) 3H-1,2,3-Triazolo[4,5-b]-pyridin-1-ol (HOAT), 7.7 mg (0.024 mMol) 1,1,3,3-Tetramethyl-2-[1H-1,2,3-triazolo[5,4-b]pyridin-1-yl]uroniumtetrafluorborat (TATU) und 18.7 mg (0.024 mMol) p-Brombenzoyl-$(Ala)_2$-Aib-$(Ala)_5$-OH in 0.3 ml N,N-Dimethylformamid wurden unter Eiskühlung und Argon 8.0 µl (0.048 mMol) Aethyldiisopropylamin zugetropft, worauf 15 Stunden bei 0° gerührt wurde. Das Reaktionsgemisch wurde eingeengt, der Rückstand wurde in Wasser und Chloroform aufgenommen, die organische Phase wurde mehrmals mit Wasser gewaschen und eingeengt, und der Rückstand wurde an 5 g Kieselgel mit Chloroform → Chloroform/Methanol (95:5) chromatographiert, worauf nach Trocknung 18.5 mg (66.1%) N-(4-Brombenzoyl)-L-alanyl-L-alanyl-2-methyl-alanyl-L-alanyl-L-alanyl-L-alanyl-L-alanyl-L-alanyl-L-alanin (12S,15S,18R)-(12,15-dimethyl-11,14,17-trioxo-10,11,12,13,-14,15,16,17,18,19,20,21-dodecahydro-1,18-etheno-dibenz[b,n][1,5,8,11]oxatriazacyclopentadecin-18-yl)-amid als weisser Festkörper erhalten wurden. MS (LDP): 1260.4 ($MH^+$).

Beispiel 5.2.1.a

In Analogie zu Beispiel 5.1.1.a wurden 40.0 mg (0.074 mMol) (12S,15S,18R)-18-Amino-12,15-dimethyl-10,11,12,13,14,15,1,17,18,19,20,21-dodecahydro-1,18-etheno-pyrido[2,3-b]benz[n][1,4,7,10]oxatriazacyclopentadecin-11,14,17-trion-trifluoracetat (1:1) in 2 ml N,N-Dimethylformamid mit 20.8 mg (0.11 mMol) Boc-L-Alanin, 31.7 mg (0.166 mMol) N-Aethyl-N'-(3-dimethylaminopropyl)-carbodiimid, 22.0 mg (0.166 mMol) 1-Hydroxybenzotriazol und 12 µl (0.074 mMol) Aethyldiisopropylamin während 20 Stunden bei 0° umgesetzt. Danach wurden nochmals 10.4 mg

(0.055 mMol) Boc-L-Alanin, 16.0 mg (0.083 mMol) N-Aethyl-N'-(3-dimethylaminopropyl)-carbodiimid, 11.0 mg (0.083 mMol) 1-Hydroxybenzotriazol und 12 µl Aethyldiisopropylamin zugegeben, worauf 8 Stunden gerührt wurde. Das Reaktionsgemisch wurde eingeengt, der Rückstand wurde in Essigester und Wasser aufgenommen, die organische Phase wurde mit Wasser gewaschen, mit $Na_2SO_4$ getrocknet und eingeengt, und der Rückstand wurde an 10 g Kieselgel mit Chloroform/Methanol (99:1) chromatographiert, worauf nach Trocknung am Hochvakuum 35.0 mg (79.7%) [(S)-1-[(12S,15S,18R)-12,15-Dimethyl-11,14,17-trioxo-10,11,12,13,14,15,16,17,18,19, 20,21-dodecahydro-1,18-etheno-pyrido[2,3-b]benz[n][1,4,7,10]oxatriazacyclopentadecin-18-ylcarbamoyl]-ethyl]-carbamidsäure-tert.butylester als weisses Pulver isoliert wurden. Smp. (Zers.) = 233-236°. $[\alpha]_D$ = -102.6° (c=0.8, Methanol).

Beispiel 5.2.2.a

Zu einer Lösung von 20.0 mg (0.034 mMol) [(S)-1-[(12S,15S,18R)-12,15-Dimethyl-11,14,17-trioxo-10,11,12,13,14,15,16,17,18,19,20,21-dodecahydro-1,18-etheno-pyrido[2,3-b]benz[n][1,4,7,10]oxatriazacyclopentadecin-18-ylcarbamoyl]-ethyl]-carbamidsäure-tert.butylester in 1.5 ml Dichlormethan wurden unter Argon bei 0° 1.5 ml Trifluoressigsäure zugegeben, worauf 3 Stunden bei 0° gerührt wurde. Das Lösungsmittel wurde am Hochvakuum entfernt, und der Rückstand wurde im Ultraschallbad in 1 ml Wasser aufgeschlämmt und dann lyophilisiert, worauf nach Trocknung am Hochvakuum 18.0 mg (87%) (S)-2-Amino-N-[(12S,15S,18R)-12,15-dimethyl-11,14,17-trioxo-10,11,12,13,14,15,16,17,18,19,20,21-dodecahydro-1,18-etheno-pyrido[2,3-b]benz[n][1,4,7,10]oxatriazacyclopentadecin-18-yl]-propionamid-trifluoracetat (1:1) als weisses Pulver erhalten wurden. Smp. (Zers.) = 265-267°. $[\alpha]_D$ = -98.2° (c=0.5, Methanol).

Beispiel 5.2.3.a

In Analogie zu Beispiel 5.1.3.a wurden 16.0 mg (26 µMol) (S)-2-Amino-N-[(12S,15S,18R)-12,15-dimethyl-11,14,17-trioxo-10,11,12,13,14,15,16,17,18,19, 20,21-dodecahydro-1,18-etheno-pyrido[2,3-b]benz[n][1,4,7,10]oxatriazacyclopentadecin-18-yl]-propionamid-trifluoracetat (1:1) in 1.5 ml N,N-Dimethylformamid mit 30.6 mg (39.0 µMol) p-Brombenzoyl-$(Ala)_2$-Aib-$(Ala)_5$-OH, 11.2 mg (58.5 µMol) N-Aethyl-N'-(3-dimethylaminopropyl)carbodiimid, 8.1 mg (60.0 µMol) 1-Hydroxybenzotriazol und 4.5 µl (20.0 µMol) N-Aethyldiisopropylamin 3 Stunden bei 0° umgesetzt. Das Reaktionsgemisch wurde am Hochvakuum eingeengt, der Rückstand wurde in Chloroform und Wasser aufgenommen, die organische Phase wurde mit Wasser gewaschen, mit $Na_2SO_4$ getrocknet und eingeengt, und der Rückstand wurde an 5 g Kieselgel mit Chloroform/Methanol (95:5) chromatographiert, worauf nach präparativer HPLC-Chromatographie (Vydac RP 18-Säule) 23.4 mg (72.0%) N-(4-Brom-benzoyl)-L-alanyl-L-alanyl-2-methyl-alanyl-L-alanyl-L-alanyl-L-alanyl-L-alanyl-L-alanin (12S,15S,18R)-(12,15-dimethyl-11,14,17-trioxo-10,11,12,13,14,15,16,17,18,19,20,21-dodecahydro-1,18-etheno-pyrido[2,3-b]benz[n][1,5,8,11]oxatriazacyclopentadecin-18-yl)-amid als weisses Pulver erhalten wurden. $[\alpha]_D$ = -57.5° (c=0.8, Methanol). MS (ISP): 1259.8 ($MH^+$; 5), 659.2 (100), 642.4 (93), 631.4 (93).

Beispiel 5.3.1.a

In Analogie zu Beispiel 5.1.1.a wurden 6.3 mg (12.2 µMol) (3S,7S,10S,13R)-13-Amino-3,7,10-trimethyl-3,4,5,6,7,8,9,10,11,12,13,14,19,20-tetradecahydro-13,15-etheno-2H-1,5,8,11-benzoxatriazacyclohexadecin-6,9,12-trion-trifluoracetat (1:1) mit 3.4 mg (18.3 µMol) Boc-L-Alanin, 3.3 mg (24.4 µMol) 3H-1,2,3-Triazolo[4,5-b]-pyridin-1-ol (HOAT), 5.89 mg (18.3 µMol) 1,1,3,3-Tetramethyl-2-[1H-1,2,3-triazolo[5,4-b]pyridin-1-yl]-uroniumtetrafluoroborat (TATU) und 7.8 µl (45.7 µMol) Aethyldiisopropylamin in 0.3 ml N,N-Dimethylformamid während 4 Stunden bei 0° umgesetzt, worauf nach Chromatographie des Rückstandes an 5 g Kieselgel mit Chloroform → Chloroform/Methanol (98:2) und Trocknung am Hochvakuum 8.2 mg (>100%) [(S)-1-[(3S,7S,10S,13R)-3,7,10-Trimethyl-6,9,12-trioxo-3,4,5,6,7,8,9,10, 11,12,13,14,19,20-tetradecahydro-13,15-etheno-2H-1,5,8,11-benzoxatriazacyclohexadecin-13-ylcarbamoyl]-ethyl]-carbamidsäure-tert.butylester als weisser Rückstand erhalten wurden. MS (LDP): 596.8 ($M+Na^+$; 100), 612.7 ($M+K^+$; 40), 357.5 (35), 495.1 (33).

Beispiel 5.3.2.a

In Analogie zu Beispiel 5.1.2.a wurden 8.2 mg (12.2 µMol) [(S)-1-[(3S,7S,10S,13R)-3,7,10-Trimethyl-6,9,12-trioxo-3,4,5,6,7,8,9,10,11,12,13,14, 19,20-tetradecahydro-13,15-etheno-2H-1,5,8,11-benzoxatriazacyclohexadecin-13-ylcarbamoyl]-ethyl]-carbamidsäure-tert.butylester in 0.3 ml Dichlormethan und 0.3 ml Trifluoressigsäure während 4 Stunden bei 0° umgesetzt, worauf nach Fallung aus Aether/Hexan (1:1) und Trocknung am Hochvakuum das entsprechende Amin als Trifluoracetat erhalten wurde. 7.2 mg (12 µl) dieses Produkts wurden in Analogie zu Beispiel 5.1.3.a mit 14.3 mg (18.3 µMol) p-Brombenzoyl-$(Ala)_2$-Aib-$(Ala)_5$-OH, 5.9 mg (18.3 µMol) 1,1,3,3-Tetramethyl-2-[1H-1,2,3-triazolo[5,4-b]pyridin-1-yl]uroniumtetrafluorborat (TATU), 3.3 mg (24.0 µMol) 3H-1,2,3-Triazolo[4,5-b]-pyridin-1-ol (HOAT) und 7.8 µl (45.0 µMol) Aethyldiisopropylamin in 0.3 ml N,N-Dimethylformamid während 6 Stunden umgesetzt. Nach

Aufarbeitung gemäss Beispiel 5.1.3.a wurde der Rückstand an 5 g Kieselgel mit Chloroform → Chloroform/Methanol chromatographiert, worauf nach zusätzlicher Reinigung mittels präparativer HPLC (Vydac RP-18) und Trocknung am Hochvakuum 5.2 mg (29.3%) N-(4-Brom-benzoyl)-L-alanyl-L-alanyl-2-methyl-alanyl-L-alanyl-L-alanyl-L-alanyl-L-alanyl-L-alanyl-L-alanin (3S,7S,10S,13R)-(3,7,10-trimethyl-6,9,12-trioxo-3,4,5,6,7,8,9,10,11,12,13,14,19,20-tetradecahydro-13,15-etheno-2H-1,5,8,11-benzoxatriazacyclohexadecin-13-yl)-amid als weisses Pulver erhalten wurden. MS (LDP): 1240.3 ($M+H^+$; 100), 374.0 (31), 391.9 (23), 411.1 (23).

Beispiel 5.4.1.a

In Analogie zu Beispiel 5.1.1.a wurden 10.0 mg (18.4 µMol) (3S,7S,10S,13R)-13-Amino-5-ethyl-3,7,10-trimethyl-3,4,5,6,7,8,9,10,11,12,13, 14,19,20-tetradecahydro-2H-1,5,8,11-benzoxatriazacyclohexadecin-6,9,12-trion-trifluoracetat (1:1) mit 5.2 mg (27.6 µMol) Boc-L-Alanin, 5.0 mg (36.8 µMol) 3H-1,2,3-Triazolo[4,5-b]-pyridin-1-ol (HOAT), 8.9 mg (27.6 µMol) 1,1,3,3-Tetramethyl-2-[1H-1,2,3-triazolo[5,4-b]pyridin-1-yl]-uroniumtetrafluorborat (TATU) und 11 µl (64.0 µMol) Aethyldiisopropylamin in 0.5 ml N,N-Dimethylformamid während 15 Stunden bei 0° umgesetzt, worauf gemäss Beispiel 5.1.1.a aufgearbeitet und gereinigt wurde. Nach Chromatographie an 10 g Kieselgel mit Chloroform → Chloroform/Methanol (95:5) und Trocknung am Hochvakuum wurden 9.5 mg (86.4%) (S)-1-[(3S,7S,10S,13R)-5-Ethyl-3,7,10-trimethyl-6,9,12-trioxo-13,15-etheno-3,4,5,6,7, 8,9,10,11,12,13,14,19,20-tetradecahydro-2H-1,5,8,11-benzoxatriazacyclohexadecin-13-ylcarbamoyl]-ethyl-carbamidsäure-tert.butylester als weisses Pulver erhalten. MS (ISP): 602.4 ($M+H^+$; 100).

Beispiel 5.4.2.a

In Analogie zu Beispiel 5.1.2.a wurden 9.5 mg (16.0 µMol) (S)-1-[(3S,7S,10S,13R)-5-Ethyl-3,7,10-trimethyl-6,9,12-trioxo-13,15-etheno-3,4,5,6,7, 8,9,10,11,12,13,14,19,20-tetradecahydro-2H-1,5,8,11-benzoxatriazacyclohexadecin-13-ylcarbamoyl]-ethyl-carbamidsäure-tert.butylester in 0.1 ml Dichlormethan und 0.1 ml Trifluoressigsäure während 2 Stunden bei 0° umgesetzt, worauf gemäss Beispiel 5.1.2.a aufgearbeitet wurde. Der Rückstand wurde an Kieselgel mit Chloroform/Methanol (95:5 → 4:1) chromatographiert, worauf nach Trocknung am Hochvakuum 9.2 mg (94.8%) (S)-2-Amino-N-[(3S,7S,10S,13R)-5-ethyl-3,7,10-trimethyl-6,9,12-trioxo-13,15-etheno-3,4,5,6,7,8,9,10,11,12,13,14,19,20-tetradecahydro-2H-1,5,8,11-benzoxatriazacyclohexadecin-13-yl]-propionamid-trifluoracetat (1:1) als weisses Pulver erhalten wurden. MS (ISP): 502.5 ($M+H^+$; 34), 267.4 (100), 289.4 (44), 211 (26), 279.3 (20).

Beispiel 5.4.3.a

In Analogie zu Beispiel 5.1.3.a wurden 9.2 mg (14.9 µMol) (S)-2-Amino-N-[(3S,7S,10S,13R)-5-ethyl-3,7,10-trimethyl-6,9,12-trioxo-13,15-etheno-3,4,5,6,7,8,9,10,11,12,13,14,19,20-tetradecahydro-2H-1,5,8,11-benzoxatriazacyclohexadecin-13-yl]-propionamid-trifluoracetat (1:1) mit 21.5 mg (27.5 µMol) p-Brombenzoyl-$(Ala)_2$-Aib-$(Ala)_5$-OH, 8.8 mg (27.5 µMol) 1,1,3,3-Tetramethyl-2-[1H-1,2,3-triazolo[5,4-b]-pyridin-1-yl]-uroniumtetrafluoroborat (TATU), 4.0 mg (29.8 µMol) 3H-1,2,3-Triazolo[4,5-b]-pyridin-1-ol (HOAT) und 8.9 µl (52.1 µMol) Aethyldiisopropylamin in 0.3 ml N,N-Dimethylformamid während 15 Stunden bei 0° umgesetzt. Anschliessend wurde gemäss Beispiel 5.1.3.a aufgearbeitet, und der Rückstand wurde an 10 g Kieselgel mit Chloroform/Methanol (99:1 → 95:5) chromatographiert, worauf nach Trocknung am Hochvakuum 14.0 mg (74%) N-(4-Brom-benzoyl)-L-alanyl-L-alanyl-2-methyl-alanyl-L-alanyl-L-alanyl-L-alanyl-L-alanyl-L-alanyl-L-alanin (3S,7S,10S,13R)-(5-ethyl-3,7,10-trimethyl-6,9,12-trioxo-13,15-etheno-3,4,5,6,7,8,9-,10,11,12,13,14,19,20-tetradecahydro-2H-1,5,8,11-benzoxatriazacyclohexaecin-13-yl)-amid als leicht beiger Feststoff erhalten wurden. MS (LDP): 1267.5 ($M+H^+$; 100), 1093.3 (22), 1191,5 (8), 1120.5 (8), 552.1 (8).

Beispiel 5.5.1.a

In Analogie zu Beispiel 5.2.1.a wurden 63 mg (0.114 mMol) (13S,16S,19R)-19-Amino-13,16-dimethyl-11,12,13,14,15,16,17,18,19,20,21,22-dodecahydro-1,19-etheno-10H-dibenz[b,o][1,5,8,11]oxatriazacyclohexadecin-12,15,18-trion-trifluoracetat (1:1) in 2 ml N,N-Dimethylformamid mit 32.0 mg (0.171 mMol) Boc-L-Alanin, 39 mg (0.256 mMol) 1-Hydroxybenzotriazol, 49 mg (0.256 mMol) N-Aethyl-N'-(3-dimethylaminopropyl)carbodiimid-hydrochlorid und 86 µl (0.53 mMol) Aethyldiisopropylamin während 24 Stunden bei 0° umgesetzt, worauf nach Chromatographie des Rückstandes an 5 g Kieselgel mit Chloroform/Methanol (95:5) ~70 mg (100%) [(S)-1-[(13S,16S,19R)-13,16-Dimethyl-12,15,18-trioxo-11,12,13,14,15,16,17,18,19, 20,22-dodecahydro-1,19-etheno-10H-dibenz[b,o][1,5,8,11]oxatriazacyclohexadecin-19-ylcarbamoyl]-ethyl]-carbamidsäure-tert.butylester als weisses Pulver erhalten wurden. Smp. 245-246°. $[\alpha]_D = +46.5°$ (c=0.2, Methanol).

Beispiel 5.5.2.a

In Analogie zu Beispiel 5.1.2.a wurden 70 mg (0.115 mMol) [(S)-1-[(13S,16S,19R)-13,16-Dimethyl-12,15,18-trioxo-11,12,13,14,15,16,17,18,19,20,22-dodecahydro-1,19-etheno-10H-dibenz[b,o][1,5,8,11]oxatriazacyclohexadecin-19-ylcarbamoyl]-ethyl]-carbamidsäure-tert.butylester in 2 ml Dichlormethan mit 2 ml Trifluoressigsäure umgesetzt, worauf nach Chromatographie des Rückstandes an 10 g Kieselgel mit Chloroform/Methanol (95:5) und Trocknung am Hochvakuum 54 mg (75.4%) (S)-2-Amino-N-[(13S,16S,19R)-13,16-dimethyl-12,15,18-trioxo-11,12,13,14,15,16,17-,18,19,20,21,22-dodecahydro-1,19-etheno-10H-dibenz[b,o][1,5,8,11]oxatriazacyclohexadecin-19-yl]-propionamid-trifluoracetat (1:1) als weisses Pulver erhalten wurden. Smp. 202-204°. MS (ISP): 508.4 ($M+H^+$; 100).

Beispiel 5.5.3.a

In Analogie zu Beispiel 5.1.3.a wurden 45.0 mg (72 µMol) (S)-2-Amino-N-[(13S,16S,19R)-13,16-dimethyl-12,15,18-trioxo-11,12,13,14,15,16,17,18,19,20, 21,22-dodecahydro-1,19-etheno-10H-dibenz[b,o][1,5,8,11]oxatriazacyclohexadecin-19-yl]-propionamid-trifluoracetat (1:1) mit 84.7 mg (0.108 mMol) p-Brombenzoyl-$(Ala)_2$-Aib-$(Ala)_5$-OH, 21.8 mg (0.162 mMol) 1-Hydroxybenzotriazol, 31.0 mg (0.162 mMol) N-Aethyl-N'-(3-dimethylaminopropyl)-carbodiimid-hydrochlorid und 18 µl (0.108 mMol) Aethyldiisopropylamin in 2.5 ml N,N-Dimethylformamid während 15 Stunden bei Raumtemperatur umgesetzt, worauf nach Chromatographie des Rückstandes an 15 g Kieselgel mit Chloroform/Methanol (95:5) und Umkristallisation aus Acetonitril 24 mg (26.1%) N-(4-Brom-benzoyl)-L-alanyl-L-alanyl-2-methyl-alanyl-L-alanyl-L-alanyl-L-alanyl-L-alanyl-L-alanyl-L-alanin (13S,16S,19R)-(13,16-dimethyl-12,15,18-trioxo-11,12,13,14,15,16,-17,18,19,20,21,22-dodecahydro-1,19-etheno-10H-dibenz[b,o][1,5,8,11]oxatriazacyclohexadecin-19-yl)-amid als weisses Pulver erhalten wurden. Smp. 285-288° (Zers.). MS (ISP): 1274.6 ($M+H^+$; 30), 665.8 (100), 686.4 (95), 707(65), 638(40).

## Patentansprüche

1. Tetrahydronaphthalinderivate der allgemeinen Formeln

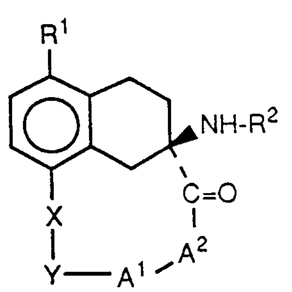

Ia und

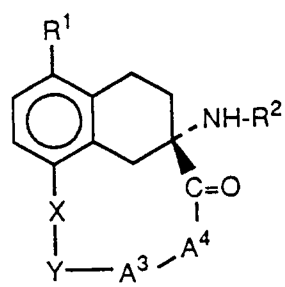

Ib

worin

$R^1$ Wasserstoff, Brom, Cyano, Formyl, Hydroxy, niederes Alkyl, niederes Alkenyl, niederes Alkinyl, niederes Alkoxy, Aryloxy, niederes Aralkoxy oder Aryl;

$R^2$ eine gegebenenfalls geschützte Kette von bis zu 20 Aminosäureresten, deren N-terminale Aminosäure eine freie oder geschützte Aminogruppe aufweist;

$A^1$, $A^2$, $A^3$ und $A^4$ je Reste von α-Aminosäuren, deren α-C-Atom, wenn es unsymmetrisch ist, in $A^1$ und $A^2$ in der L-Konfiguration und in $A^3$ und $A^4$ in der D-Konfiguration vorliegt;

X Sauerstoff oder Schwefel;

Y einen Rest der Formel

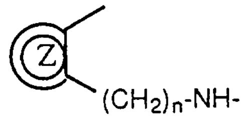
(a)

oder

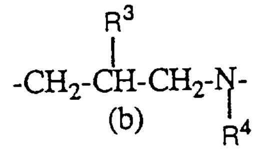
(b)

n die Zahl 0 oder 1;
$R^3$ und $R^4$ je Wasserstoff oder niederes Alkyl; und
Z zusammen mit den beiden C-Atomen einen gegebenenfalls substituierten Benzol-, Furan-, Thiophen-, Pyridin- oder Pyrimidinring bedeuten;

sowie Salze davon.

**2.** Verbindungen nach Anspruch 1, worin $R^1$ in der Bedeutungsmöglichkeit "Aryl" durch niederes Alkyl oder niederes Alkoxy mono- oder disubstituiertes oder durch niederes Alkylendioxy substituiertes Phenyl bedeutet.

**3.** Verbindungen nach Anspruch 1 oder 2, worin $R^2$ 9 bis 15 Aminosäurereste enthält.

**4.** Verbindungen nach Anspruch 3, worin $R^2$ 9, 12 oder 15 Aminosäurereste enthält.

**5.** Verbindungen nach einem der Ansprüche 1 bis 4, worin $R^2$ Reste von $\alpha$-Aminosäuren enthält, deren $\alpha$-C-Atom, wenn es asymmetrisch ist, in der L-Konfiguration vorliegt.

**6.** Verbindungen nach Anspruch 5, worin $R^2$ Reste von L-Alanin, L-Glutamin, L-Glutaminsäure, L-Isoleucin, L-Leucin, L-Lysin oder 2-Amino-2-methylpropionsäure enthält.

**7.** Verbindungen nach einem der Ansprüche 1 bis 6, worin $A^1$ bzw. $A^3$ einen Rest von L- bzw. D-Alanin, Asparagin, Asparaginsäure, Glutamin, Glutaminsäure oder Lysin oder von 2-Amino-2-methylpropionsäure und $A^2$ bzw. $A^4$ einen Rest von L- bzw. D-Alanin, Asparagin, Asparaginsäure, Glutamin, Glutaminsäure oder Lysin bedeuten.

**8.** Verbindungen nach Anspruch 7, worin $A^1$ bzw. $A^3$ L- bzw. D-Alanyl und $A^2$ bzw. $A^4$ L- bzw. D-Alanyl bedeuten.

**9.** Verbindungen nach einem der Ansprüche 1 bis 8, worin Y einen Rest der Formel (c), n die Zahl 0 und Z zusammen mit den beiden C-Atomen einen Benzolring bedeuten.

**10.** N-(4-Brom-benzoyl)-L-alanyl-L-alanyl-2-methyl-alanyl-L-alanyl-L-alanyl-L-alanyl-L-alanyl-L-alanyl-L-alanin (12S,15S,18R)-(12,15-dimethyl-11,14,17-trioxo-10,11,12,13,14,15,16,17,18,19,20,21-dodecähydro-1,18-etheno-dibenz[b,n][1,5,8,11]oxatriazacyclopentadecin-18-yl)-amid,

N-(4-Brom-benzoyl)-L-alanyl-L-alanyl-2-methyl-alanyl-L-alanyl-L-alanyl-L-alanyl-L-alanyl-L-alanyl-L-alanin (12S,15S,18R)-(12,15-dimethyl-11,14,17-trioxo-10,11,12,13,14,15,16,17,18,19,20,21-dodecahydro-1,18-etheno-pyrido[2,3-b]benz[n][1,5,8,11]oxatriazacyclopentadecin-18-yl)-amid;
N-(4-Brom-benzoyl)-L-alanyl-L-alanyl-2-methyl-alanyl-L-alanyl-L-alanyl-L-alanyl-L-alanyl-L-alanyl-L-alanin (3S,7S,10S,13R)-(3,7,10-trimethyl-6,9,12-trioxo-3,4,5,6,7,8,9,10,11,12,13,14,19,20-tetradecahydro-13,15-etheno-2H-1,5,8,11-benzoxatriazacyclohexadecin-13-yl)-amid;
N-(4-Brom-benzoyl)-L-alanyl-L-alanyl-2-methyl-alanyl-L-alanyl-L-alanyl-L-alanyl-L-alanyl-L-alanyl-L-alanin (3S,7S,10S,13R)-(5-ethyl-3,7,10-trimethyl-6,9,12-trioxo-13,15-etheno-3,4,5,6,7,8,9,10,11,12,13,14,19,20- tetradecahydro-2H-1,5,8,11-benzoxatriazacyclohexadecin-13-yl)-amid; und
N-(4-Brom-benzoyl)-L-alanyl-L-alanyl-2-methyl-alanyl-L-alanyl-L-alanyl-L-alanyl-L-alanyl-L-alanyl-L-alanin (13S,16S,19R)-(13,16-dimethyl-12,15,18-trioxo-11,12,13,14,15,16,17,18,19,20,21,22-dodecahydro-1,19-etheno-10H-dibenz[b,o][1,5,8,11]oxatriazacyclohexadecin-19-yl)-amid.

**11.** Verfahren zur Herstellung von Verbindungen nach einem der Ansprüche 1 bis 10, dadurch gekennzeichnet, dass man

a) eine Verbindung der allgemeinen Formel

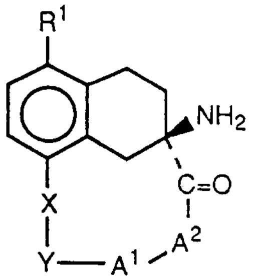

IIa bzw.

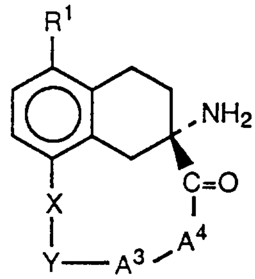

IIb

worin $R^1$, $A^1$, $A^2$, $A^3$, $A^4$, X und Y die in Anspruch 1 angegebene Bedeutung besitzen, in einem Schritt oder, vorzugsweise, in Etappen mit einer gegebenenfalls geschützten Kette von bis zu 20 Aminosäuren, deren N-terminale Aminosäure eine freie oder geschützte Aminogruppe aufweist, kuppelt; oder

b) eine Verbindung der allgemeinen Formel

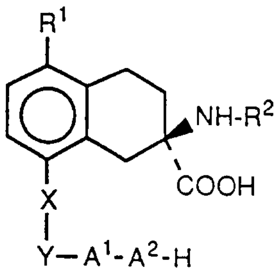

IIIa bzw.

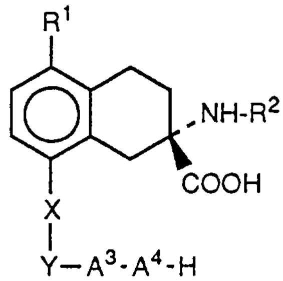

IIIb

worin $R^1$, $R^2$, $A^1$, $A^2$, $A^3$, $A^4$, X und Y die in Anspruch 1 angegebene Bedeutung besitzen, cyclisiert; oder

c) aus einer Verbindung der Formel Ia bzw. Ib, welche mindestens eine Schutzgruppe enthält, die Schutzgruppe(n) abspaltet; oder

d) eine Verbindung der Formel Ia bzw. Ib, welche ein basisches bzw. ein saures Zentrum enthält, mittels einer Säure bzw. einer Base in ein Salz überführt.

**12.** Verbindungen der in Anspruch 11 definierten allgemeinen Formeln IIa, IIb, IIIa und IIIb.

**13.** Verbindungen der allgemeinen Formeln

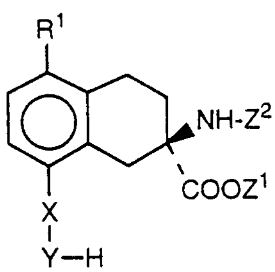

IVa bzw.

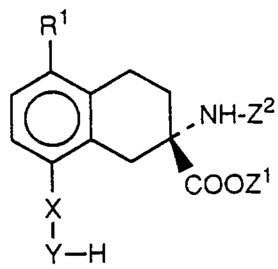

IVb

worin $R^1$ und Y die in Anspruch 1 angegebene Bedeutung besitzen und $Z^1$ eine Carboxylschutzgruppe und $Z^2$ eine Aminoschutzgruppe bedeuten.

**14.** Verbindungen gemäss Anspruch 13, worin $Z^1$ Methyl, tert.Butyl, Benzyl, Trimethylsilyläthyl oder Pentafluorphenyl und $Z^2$ Benzyloxycarbonyl, tert.Butyloxycarbonyl oder Fluoren-9-ylmethoxycarbonyl bedeuten.

15. Verwendung von Verbindungen gemäss einem der Ansprüche 1 bis 10 als "Research Tools" zur Ermittlung biologisch aktiver Peptidsequenzen.

Fig. 1

CD Kurven gemessen in Wasser/TFE (1:1)

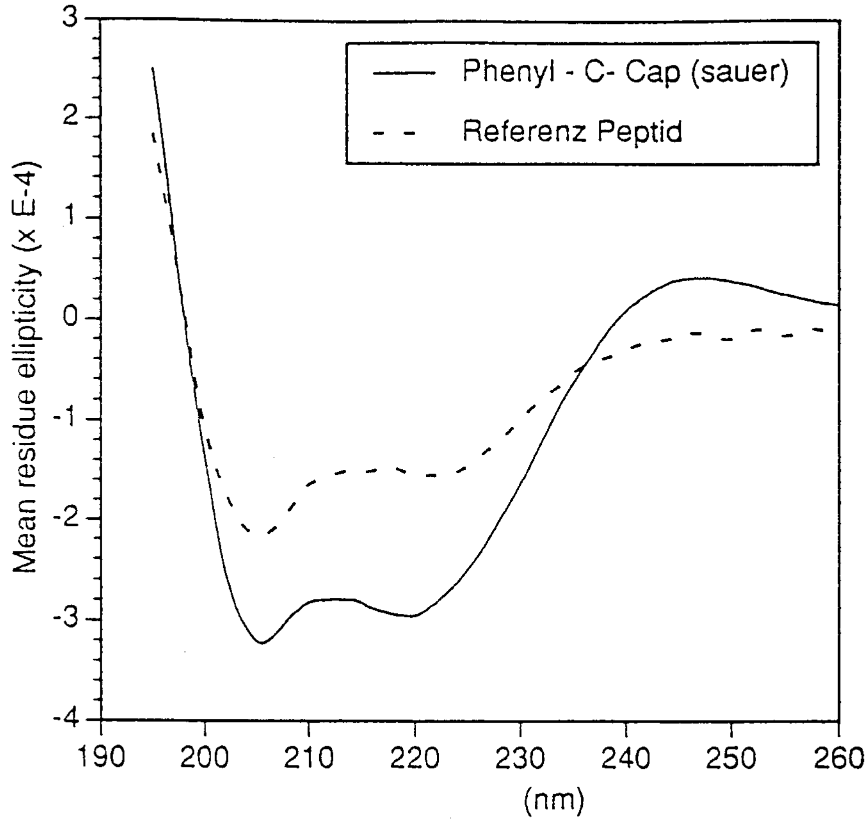


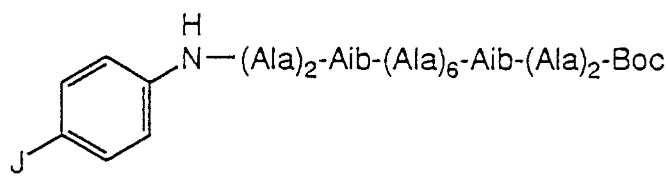

Referenz
Peptid
50%helikal
neutral

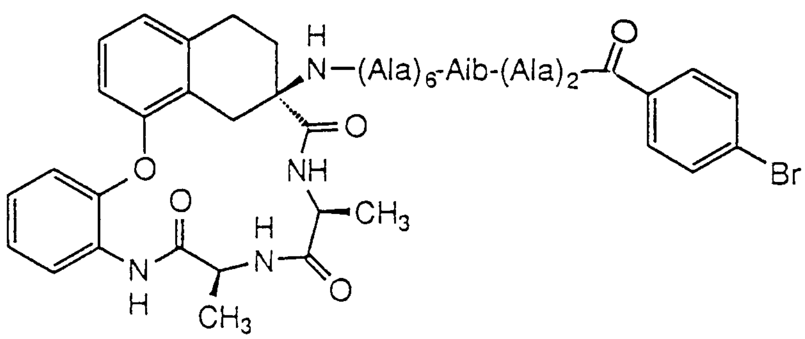

Phenyl -
C-Cap
92%helikal
sauer
81%helikal
neutral

Europäisches Patentamt

# EUROPÄISCHER RECHERCHENBERICHT

Nummer der Anmeldung
EP 96 10 2256

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | EP-A-0 592 791 (F. HOFFMANN-LA ROCHE AG)<br>* das ganze Dokument *<br>--- | | C07K5/10<br>C07K7/06<br>C07K7/08 |
| A | TETRAHEDRON, (INCL. TETRAHEDRON REPORTS), Bd. 49, Nr. 17, 23.April 1993, OXFORD GB, Seiten 3479-3488, XP002005833<br>J.F. CALLAHAN ET AL.: "The Use of gamma-Turn mimetics to Define Peptide Secondary structure"<br>* das ganze Dokument *<br>--- | | |
| A | JOURNAL OF MEDICINAL CHEMISTRY, Bd. 35, Nr. 21, 16.Oktober 1992, WASHINGTON US, Seiten 3970-3972, XP002005834<br>J.F. CALLAHAN ET AL.: "Design and Synthesis of a C7 miemtic for the Predicted gamma-Turn Conformation Found in Several Constrained RGD Antagonists"<br>* das ganze Dokument *<br>--- | | RECHERCHIERTE SACHGEBIETE (Int.Cl.6) |
| P,X | EP-A-0 640 618 (HOFFMANN LA ROCHE) 1.März 1995<br>* Ansprüche; Beispiele *<br>----- | 1-10,15 | C07K |

Der vorliegende Recherchenbericht wurde für alle Patentansprüche erstellt

| Recherchenort | Abschlußdatum der Recherche | Prüfer |
|---|---|---|
| DEN HAAG | 17.Juni 1996 | Fuhr, C |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)